# EUROPEAN PATENT SPECIFICATION

(11) **EP 1 299 753 B1**
(45) Date of publication and mention of the grant of the patent: **12.05.2004**
(21) Application number: 01945203.6
(22) Date of filing: 28.05.2001
(51) Int. Cl.: G02B 1/04, A61L 27/34

(54) **COATED ARTICLES**
BESCHICHTETE GEGENSTÄNDE
ARTICLES REVETUS

(30) Priority: 30.05.2000 EP 00111526; 24.08.2000 US 228022 P
(43) Date of publication of application: 09.04.2003
(73) Proprietor: Novartis AG, 4056 Basel (CH); Novartis Pharma GmbH, 1230 Wien (AT)
(72) Inventor: CHABRECEK, Peter, CH-4125 Riehen (CH); LOHMANN, Dieter, CH-4142 Münchenstein (CH); LEUKEL, Jörg, D-79110 Freiburg (DE); WINTERTON, Lynn, Cook, Alpharetta, GA 30201 (US); QIU, Yongxing, Duluth, GA 30097 (US); LALLY, John, Martin, Lilburn, GA 30047 (US)
(74) Representative: Gros, Florent
(86) International application number: PCT/EP2001/006082
(87) International publication number: WO 2001/092924

(56) References cited:
- EP-A- 1 095 711
- EP-A- 1 095 966
- WO-A-96/20796
- WO-A-96/20919
- WO-A-99/35520
- WO-A-99/57581
- US-A- 5 807 636

## Description

The present invention relates to coated articles such as biomedical articles, especially contact lenses, which are at least partly coated with a hydrophilic polymer, and to a process for the manufacture of said coated articles.

A variety of different types of processes for preparing hydrophilic polymeric coatings on an "inert" hydrophobic substrate have been disclosed in the prior art. For example, WO 99/57581 discloses to first of all provide the article surface with covalently bound photoinitiator molecules, coating the modified surface with a layer of a polymerizable macromonomer and then subjecting it to a heat or radiation treatment whereby the macromonomer is graft polymerized thus forming the novel article surface. The covalent binding of the photoinitiator molecules to the article surface is created by first subjecting the article surface to a plasma treatment thereby providing the surface with functional groups, and then reacting said functional groups with coreactive groups of a functional photoinitiator.

A plasma treatment requires a considerable investment in equipment and is furthermore difficult to be integrated in an automated production process. For example, a plasma treatment requires that the article to be treated is dry before exposure to the plasma. Thus, a polymeric article such as a contact lens that is wet from prior hydration or extraction must be dried previously, thereby adding time in the overall lens production process as well as imposing added costs of obtaining a drying equipment.

Therefore, it would be highly desirable to modify the surface functionalization step of the process disclosed in WO 99/57581 such that the plasma treatment is avoided and replaced by a technique which is easy to perform with standard equipment and which is thus more feasible for an automated production process.

Surprisingly, it has now been found, that hydrophobic articles may be readily functionalized by adding at least one polyelectrolyte or preferably a bilayer of functional polyelectrolytes to the article surface. The functional groups of the polyelectrolytes that are adsorbed and/or heteropolarly bound on the surface then may be used for the covalent attachment of polymerization initiators which in turn may initiate the graft polymerization of suitable hydrophilic monomers or macromonomers onto the article surface.

The present invention therefore in one aspect relates to a composite material comprising
(a) an inorganic or organic bulk material having attached to its surface a polyionic material that comprises covalently bound initiator moieties for radical polymerization; and
(b) a hydrophilic surface coating obtainable by applying one or more different ethylenically unsaturated hydrophilic monomers or macromonomers to the bulk material surface provided with the initiator radicals and polymerizing said monomers or macromonomers.

The bulk material underlying the composite materials of the invention is preferably a material that is devoid of ionic groups such as cationic or anionic groups. Accordingly, the surface of the preferred bulk materials is also devoid of ionic groups such as carboxy, sulfo, amino and the like groups and is thus substantially free from ionic charges.

Examples of suitable bulk materials are quartz, ceramics, glasses, silicate minerals, silica gels, metals, metal oxides, carbon materials such as graphite or glassy carbon, natural or synthetic organic polymers, or laminates, composites or blends of said materials, in particular natural or synthetic organic polymers or modified biopolymers which are known in large number. Some examples of polymers are polyaddition and polycondensation polymers (polyurethanes, epoxy resins, polyethers, polyesters, polyamides and polyimides); vinyl polymers (polyacrylates, polymethacrylates, polyacrylamides, polymethacrylamides, polystyrene, polyethylene and halogenated derivatives thereof, polyvinyl acetate and polyacrylonitrile); or elastomers (silicones, polybutadiene and polyisoprene).

A preferred group of materials to be coated are those being conventionally used for the manufacture of biomedical devices, e.g. contact lenses, in particular contact lenses for extended wear, which are not hydrophilic per se. Such materials are known to the skilled artisan and may comprise for example polysiloxanes, perfluoroalkyl polyethers, fluorinated poly(meth)acrylates or equivalent fluorinated polymers derived e.g. from other polymerizable carboxylic acids, polyalkyl (meth)acrylates or equivalent alkylester polymers derived from other polymerizable carboxylic acids, or fluorinated polyolefines, such as fluorinated ethylene or propylene, for example tetrafluoroethylene, preferably in combination with specific dioxols, such as perfluoro-2,2-dimethyl-1,3-dioxol. Examples of suitable bulk materials are e.g. Lotrafilcon A, Neofocon, Pasifocon, Telefocon, Silafocon, Fluorsilfocon, Paflufocon, Silafocon, Elastofilcon, Fluorofocon or Teflon AF materials, such as Teflon AF 1600 or Teflon AF 2400 which are copolymers of about 63 to 73 mol % of perfluoro-2,2-dimethyl-1,3-dioxol and about 37 to 27 mol % of tetrafluoroethylene, or of about 80 to 90 mol % of perfluoro-2,2-dimethyl-1,3-dioxol and about 20 to 10 mol % of tetrafluoroethylene.

Another group of preferred materials to be coated are amphiphilic segmented copolymers comprising at least one hydrophobic segment and at least one hydrophilic segment which are linked through a bond or a bridge member. Examples are silicone hydrogels, for example those disclosed in PCT applications WO 96/31792 and WO 97/49740 which are herewith incorporated by reference.

A particular preferred group of bulk materials comprises organic polymers selected from polyacrylates, polymethacrylates, polyacrylamides, poly(N,N-dimethylacrylamides), polymethacrylamides, polyvinyl acetates, polysiloxanes, perfluoroalkyl polyethers, fluorinated polyacrylates or -methacrylates and amphiphilic segmented copolymers comprising at least one hydrophobic segment, for example a polysiloxane or perfluoroalkyl polyether segment or a mixed polysiloxane/perfluoroalkyl polyether segment, and at least one hydrophilic segment, for example a polyoxazoline, poly(2-hydroxyethylmethacrylate), polyacrylamide, poly(N,N-dimethylacrylamide), polyvinylpyrrolidone polyacrylic or polymethacrylic acid segment or a copolymeric mixture of two or more of the underlying monomers.

The material to be coated may also be any blood-contacting material conventionally used for the manufacture of renal dialysis membranes, blood storage bags, pacemaker leads or vascular grafts. For example, the material to be modified on its surface may be a polyurethane, polydimethylsiloxane, polytetrafluoroethylene, polyvinylchloride, Dacron™ or Silastic™ type polymer, or a composite made therefrom.

Moreover, the material to be coated may also be an inorganic or metallic base material without suitable reactive groups, e.g. ceramic, quartz, or metals, such as silicon or gold, or other polymeric or non-polymeric substrates. E.g. for implantable biomedical applications, ceramics are very useful. In addition, e.g. for biosensor purposes, hydrophilically coated base materials are expected to reduce nonspecific binding effects if the structure of the coating is well controlled. Biosensors may require a specific carbohydrate coating on gold, quartz, or other non-polymeric substrates.

The form of the material to be coated may vary within wide limits. Examples are particles, granules, capsules, fibres, tubes, films or membranes, preferably moldings of all kinds such as ophthalmic moldings, for example intraocular lenses, artificial cornea or in particular contact lenses.

The polyionic material being attached to the bulk material surface may consist of one single ionic polymer, for example of a polyanionic or polycationic material as described below.

Preferably, the polyionic material includes at least one bilayer, the bilayer comprising a first ionic polymer and a second ionic polymer having charges opposite of the charges of the first ionic polymer,

A suitable bilayer on the bulk material comprises a first and second ionic polymer having opposite charges, wherein "first ionic polymer" indicates the polymer that is first of all applied to the article surface, and "second ionic polymer" indicates the polymer that is applied to the article surface after it has already been modified with the first ionic polymer. The bulk material may comprise one or more than one bilayers, for example from 1 to 25 bilayers containing the same or different ionic polymers in each case, preferably from 1 to 20 bilayers, more preferably 1 to 10 bilayers, even more prefereably 1 to 5 bilayers and in particular just one bilayer.

The first ionic polymer may be cationic or anionic, preferably anionic. A suitable anionic polymer is, for example, a synthetic polymer, biopolymer or modified biopolymer comprising carboxy, sulfo, sulfato, phosphono or phosphate groups or a mixture thereof, or a salt thereof, for example a biomedical acceptable salt and especially an ophthalmically acceptable salt thereof. Anionic polymers comprising carboxy groups or a suitable salt thereof are preferred.

Examples of synthetic anionic polymers are: a linear polyacrylic acid (PAA), a branched polyacrylic acid, for example a Carbophil® or Carbopol® type from Goodrich Corp., a polymethacrylic acid (PMA), a polyacrylic acid or polymethacrylic acid copolymer, for example a copolymer of acrylic or methacrylic acid and a further vinylmonomer, for example acrylamide, N,N-dimethyl acrylamide or N-vinylpyrrolidone, a maleic or fumaric acid copolymer, a poly(styrenesulfonic acid) (PSS), a polyamido acid, for example a carboxy-terminated polymer of a diamine and a di- or polycarboxylic acid, for example carboxy-terminated Starburst™ PAMAM dendrimers (Aldrich), a poly(2-acrylamido-2-methylpropanesulfonic acid) (poly-(AMPS)), or an alkylene polyphosphate, alkylene polyphosphonate, carbohydrate polyphosphate or carbohydrate polyphosphonate, for example a teichoic acid.

Examples of anionic biopolymers or modified biopolymers are: hyaluronic acid, glycosaminoglycanes such as heparin or chondroitin sulfate, fucoidan, poly-aspartic acid, poly-glutamic acid, carboxymethyl cellulose, carboxymethyl dextranes, alginates, pectins, gellan, carboxyalkyl chitins, carboxymethyl chitosans, sulfated polysaccharides.

A preferred anionic polymer is a linear or branched polyacrylic acid or an acrylic acid copolymer. A more preferred anionic polymer is a linear or branched polyacrylic acid. A branched polyacrylic acid in this context is to be understood as meaning a polyacrylic acid obtainable by polymerizing acrylic acid in the presence of suitable (minor) amounts of a di- or polyvinyl compound.

A suitable cationic polymer as part of the bilayer is, for example, a synthetic polymer, biopolymer or modified biopolymer comprising primary, secondary or tertiary amino groups or a suitable salt thereof, preferably an ophthalmically acceptable salt thereof, for example a hydrohalogenide such as a hydrochloride thereof, in the backbone or as substituents. Cationic polymers comprising primary or secondary amino groups or a salt thereof are preferred.

Examples of synthetic cationic polymers are:
(i) a polyallylamine (PAH) homo- or copolymer, optionally comprising modifier units;
(ii) a polyethyleneimine (PEI);
(iii) a polyvinylamine homo- or copolymer, optionally comprising modifier units;
(iv) a poly(vinylbenzyl-tri-C₁-C₄-alkylammonium salt), for example a poly(vinylbenzyl-trimethyl ammoniumchloride);
(v) a polymer of an aliphatic or araliphatic dihalide and an aliphatic N,N,N',N'-tetra-C₁-C₄-alkyl-alkylenediamine, for example a polymer of (a) propylene-1,3-dichloride or -dibromide or p-xylylene dichloride or dibromide and (b) N,N,N',N'-tetramethyl-1,4-tetramethylene diamine;
(vi) a poly(vinylpyridin) or poly(vinylpyridinium salt) homo- or copolymer;
(vii) a poly (N,N-diallyl-N,N-di-C₁-C₄-alkyl-ammoniumhalide) comprising units of formula 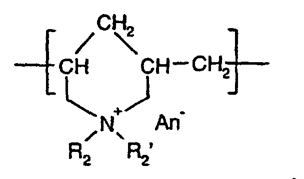 wherein R₂ and R₂' are each independently C₁-C₄-alkyl, in particular methyl, and An⁻ is a, for example, a halide anion such as the chloride anion;
(viii) a homo- or copolymer of a quatemized di-C₁-C₄-alkyl-aminoethyl acrylate or methacrylate, for example a poly(2-hydroxy-3-methacryloylpropyltri-C₁-C₂-alkylammonium salt) homopolymer such as a a poly(2-hydroxy-3-methacryloylpropyltri-methylammonium chloride), or a quatemized poly(2-dimethylaminoethyl methacrylate or a quatemized poly(vinylpyrrolidone-co-2-dimethylaminoethyl methacrylate);
(ix) POLYQUAD® as disclosed in EP-A-456,467; or
(x) a polyaminoamide (PAMAM), for example a linear PAMAM or a PAMAM dendrimer such as a amino-terminated Starbust™ PAMAM dendrimer (Aldrich).

The above mentioned polymers comprise in each case the free amine, a suitable salt thereof, for example a biomedically acceptable salt or in particular an ophthalmically acceptable salt thereof, as well as any quatemized form, if not specified otherwise.

Suitable comonomers optionally incorporated in the polymers according to (i), (iii), (vi) or (viii) above are, for example, acrylamide, methacrylamide, N,N-dimethyl acrylamide, N-vinylpyrrolidone and the like.

Suitable modifier units of the polyallylamine (i) are, for example, of formula 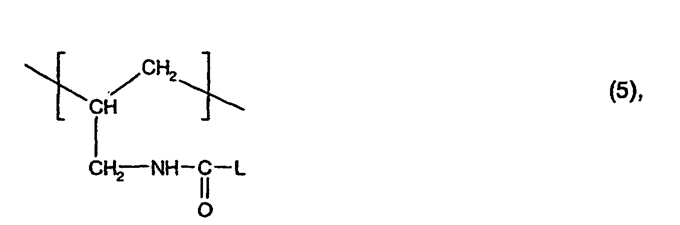 wherein L is C₂-C₆-alkyl which is substituted by two or more same or different substituents selected from the group consisting of hydroxy, C₂-C₅-alkanoyloxy and C₂-C₅-alkylaminocarbonyloxy.

L is preferably linear C₃-C₆-alkyl, more preferably linear C₄-C₅-alkyl, and most preferably n-pentyl which is in each case substituted as defined above.

Suitable substituents of the alkyl radical L are -OH, a radical -O-C(O)-R₂₉ and/or a radical -O-C(O)-NH-R₂₉' wherein R₂₉ and R₂₉' are each independently of the other C₁-C₄-alkyl, preferably methyl, ethyl or n- or iso-propyl, and more preferably methyl or ethyl.

Preferred substituents of the alkyl radical L are hydroxy, acetyloxy, propionyloxy, methylaminocarbonyloxy or ethylaminocarbonyloxy, especially hydroxy, acetyloxy or propionyloxy and in particular hydroxy.

A preferred embodiment relates to polyallyl amines comprising units of the above formula (5), wherein L is a radical of formula 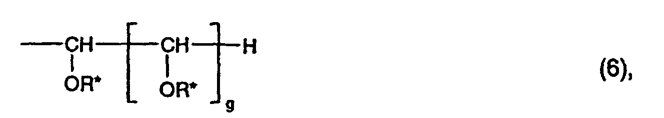 wherein g is 1, 2, 3, 4 or 5, preferably 3 or 4 and in particular 4, each R* is independently hydrogen or a radical -C(O)-R₂₉ or -C(O)-NH-R₂₉', and for R₂₉ and R₂₉' the above meanings and preferences apply. L is even more preferred a radical of the above formula (6) wherein g is 3 or 4, in particular 4, and each group -OR* independently is hydroxy or hydroxy which is partly or completely acetylated, in particular hydroxy. Particular preferred radicals L are 1,2,3,4,5-pentahydroxy-n-pentyl or 1,2,3,4,5-pentahydroxy-n-pentyl wherein the hydroxy groups are partly or completely acetylated.

The modified polyallylamines of the invention are derivatives of a polyallyl amine that, based on the number of amino groups of the polyallyl amine, comprise from about 1 to 99 %, preferably from 10 to 80 %, more preferably, from 15 to 75 %, even more preferably 20 to 70 % and in particular 40 to 60 %, of units of formula (5).

The term units of formula (5) or of another formula number below is always to be understood as encompassing one or more different species falling under the respective formula. Preferably the term means one single species. In addition, the polyallylamine may contain further modifier units, for example those disclosed in EP-A-1002807, formula (2a)-(2d).

A preferred polyallylamine according to the invention is a polyallylamine without modifier units or a polyallylamine having from 10 to 80 % of units of the above formula (5) based on the number of amino groups of the polyallyl amine. A particular preferred polyallylamine according to the invention is a polyallylamine without modifier units or a polyallylamine having from 15 to 75 %, based on the number of amino groups of the polyallyl amine, of units of the above formula (5) wherein L is 1,2,3,4,5-pentahydroxy-n-pentyl.

Suitable modifier units of the polyvinylamine (iii) are, for example, of formula 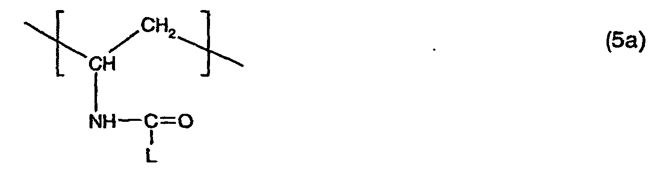 wherein for L the above-given meanings and preferences apply.

A suitable polyvinylamine copolymer is, for example, a copolymer comprising vinylamine units and units derived from another hydrophilic comonomer, for example from acrylamide, N,N-dimethyl acrylamide, N-vinylpyrrolidone or the like.

Examples of cationic biopolymers or modified biopolymers are: basic peptides, proteins or glucoproteins, for example a poly-ε-lysine, albumin or collagen, aminoalkylated polysaccharides, for example a chitosan, aminodextranes.

A preferred cationic polymer forming the bilayer that is attached to the bulk material is a polyallylamine homopolymer; a polyallylamine comprising modifier units of the above formula (1); a polyvinylamine homo- or -copolymer or a polyethyleneimine homopolymer, in particular a polyallylamine or polyethyleneimine homopolymer or a poly(vinylamine-co-acrylamid) copolymer.

The molecular weight of the anionic and cationic polymers used to prepare the bilayers may vary within wide limits depending on the desired characteristics such as adhesion on the bulk material, coating thickness and the like. In general, a weight average molecular weight of from about 5000 to about 5000000, preferably from 10000 to 1000000, more preferably 15000 to 500000, even more preferably from 20000 to 200000 and in particular from 40000 to 150000, has proven as valuable both for the anionic and cationic polymer forming the bilayer.

The anionic and cationic polymers used to prepare the bilayers are in general water-soluble. The anionic and cationic polymers forming the bilayers of the invention are known and the majority of them is commercially available, or they may be prepared according to methods known in the art. Polyallylamines comprising modifier units are known, for example, from EP-A-1 002807.

The formation and application of the bilayers on the bulk material surface may be accomplished according to processes known per se. For example, the bulk material is immersed in a solution of the anionic and cationic polymer, or one or more layers each of the anionic and cationic polymer are successively deposited on the modified bulk material surface, for example, by dipping, spraying, printing, spreading, pouring, rolling, spin coating or vacuum vapor deposition, spraying or particularly dipping being preferred. Following the deposition of one ionic polymer the bulk material may be rinsed or dried before the deposition of the next ionic polymer having opposite charges. However, it is preferred to omit a rinsing or drying step between the attachment of the first and second ionic polymer.

A preferred dip method involves the steps of (i) applying a coating of a first ionic polymer, for example of a cationic or preferably of an anionic polymer, to the bulk material by immersing the bulk material in a solution of the first ionic polymer; (ii) optionally, rinsing the bulk material by immersing it in a rinsing solution; (iii) optionally, drying said bulk material; and (iv) applying a coating of a second ionic polymer having charges opposite of the charges of the first ionic polymer, for example an anionic or preferably a cationic polymer, to the bulk material by immersing the bulk material in a solution of the second ionic polymer. A more preferred dip method involves the steps of applying a coating of the first and second ionic polymer by immersing the bulk material successively in a solution each of the first and second ionic polymer without a rinsing or drying step in between. A further dip method involves immersing the bulk material in a solution comprising both the anionic and cationic polymer.

The dip solutions of the anionic and cationic polymer in general comprise the respective polymer diluted in one or more different solvents. Suitable solvents are, for example, water or an aqueous solution comprising a water-miscible organic solvent, for example a C₁-C₄-alkanol such as methanol or ethanol; the preferred solvent is pure water. The aqueous solutions of the cationic or anionic polymer advantageously each have a slightly acidic pH value, for example a pH from about 2 to about 5 and preferably from about 2.5 to about 4.5. The concentration of the dip solutions may vary within wide limits depending, for example, on the particular ionic polymer involved. However, it is generally preferred to formulate relatively dilute solutions of the ionic polymers. A preferred anionic or cationic polymer concentration is from about 0.0001 to about 0.25 weight percent, more preferably from 0.0005 to 0.15 weight percent and in particular from 0.001 to 0.1 percent by weight, relative to the total weight of the solution.

A suitable rinsing solution, if used, is preferably an aqueous solution, in particular an aqueous solution buffered at a pH of about 2 to about 7, more preferably from 2 to 5 and even more preferably from 2.5 to 4.5.

Partial drying or removal of excess rinsing solution from the surface between solution applications, if applicable, may be accomplished by a number of means known in the art. While the bulk material may be partially dried by merely allowing the lens to remain in an air atmosphere for a certain period of time, it is preferable to accelerate the drying by application of a mild stream of air to the surface. The flow rate may be adjusted as a function of the strength of the material being dried and the mechanical fixturing of the material. It should be noted that there is no requirement to completely dry the bulk material. The "partial drying" step, as used herein, refers to a removal of droplets of solution which cling to the lens surface, rather than a desiccation of the lens. Thus, it is preferred to dry only to the extent that any water or solution film on the surface is removed.

The thickness of the coating may be adjusted by addition of one or more salts, such as sodium chloride, to the ionic polymer solution. A preferred salt concentration is about 0.1 to about 2.0 weight percent. As the salt concentration is increased, the polyelectrolyte takes on a more globular conformation. However, if the concentration is raised too high, the polyelectrolyte will not deposit well, if at all, on the lens surface. A more preferred salt concentration is about 0.7 to about 1.3 weight percent.

The bilayer formation process may be repeated a plurality of times, for example from 1 to 24 times, preferably from 1 to 14 times, more preferably from 1 to 9 times; according to one embodiment just one bilayer is deposited.
The immersion time for each of the coating and optional rinsing steps may vary depending on a number of factors. In general a rinsing time of from about 30 seconds to about 30 minutes, prefereably from 1 to 20 minutes, more preferably 1 to 10 minutes and in particular 1 to 6 minutes has proven as valuable. The immersion in the polymer solutions takes place, for example, at room temperature or at elevated temperature, preferably at room temperature, for example at a temperature of from 15 to 30°C. Following the dipping steps the bulk material may be subjected to a heat treatment in order to compact or stabilize the bilayer(s) on the bulk material surface.

instead of coating the bulk material by means of a dip technique, said coating may also take place using spray coating techniques, wherein the above given conditions and features concerning solvents, concentrations, presence of salts, pH, temperature, number and sequence of coating steps, optional rinsing or drying steps apply accordingly. Spray coating technique in this context comprises any known process in the art including, for example, conventional techniques of applying a fluid, or techniques using ultrasonic energy, or electrostatic spray coating techniques. In addition a mixture of dip and spray techniques may also be employed.

In addition, if the polyionic material on the material surface consists of one single ionic polymer only, said ionic polymer may be applied to the surface as described above, in particular by dipping or spraying.

According to the above-mentioned methods bulk materials are obtained that comprise one polyelectrolyte or preferably one or more bilayers of polyelectrolytes adsorbed and/or heteropolarly bound on the surface. Due to this modification the surface is provided with functional groups, for example with carboxy, sulfone, sulfato, phosphono or phosphato groups or with primary, secondary or tertiary amine groups; said functional groups, preferably the carboxy groups or in particular the primary or secondary amino groups, may be further reacted with a functional initiator for radical polymerization.

Polymerization initiators bound to the polyionic material that is attached to the bulk material surface are typically those that are initiating a radical polymerization of ethylenically unsaturated compounds. The radical polymerization may be induced thermally, or preferably by irradiation.

Suitable thermal polymerization initiators are known to the skilled artisan and comprise for example peroxides, hydroperoxides, azo-bis(alkyl- or cycloalkylnitriles), persulfates, percarbonates or mixtures thereof. An example for a functionalized thermal initiator is 4,4'-azo-bis(4-cyanovaleric acid) or derivatives thereof.

Initiators for the radiation-induced polymerization are particularly functional photoinitiators having a photoinitiator part and in addition a functional group that is coreactive with functional groups of the bilayers, particularly with amino or carboxy groups. The photoinitiator part may belong to different types, for example to the thioxanthone type and preferably to the benzoin type. Suitable functional groups that are coreactive with the bilayers attached to the surface of the bulk material are for example a carboxy, hydroxy, epoxy or particularly an isocyanato group.

Preferred polymerization initiators for use in the present invention are the photoinitiators of formulae (I) and (Ia) as disclosed in US patent No. 5,527,925, those of the formula (I) as disclosed in PCT application WO 96/20919, or those of formulae II and III including formulae IIa-IIy and IIIg as disclosed in EP-A-0281941, particularly formulae IIb, IIi, IIm, IIn, IIp, IIr, IIs, IIx and IIIg therein. The respective portion of said three documents including the definitions and preferences given for the variables in said formulae are herewith included by reference.

The polymerization initiator moieties are preferably derived from a functional photoinitiator of the formula 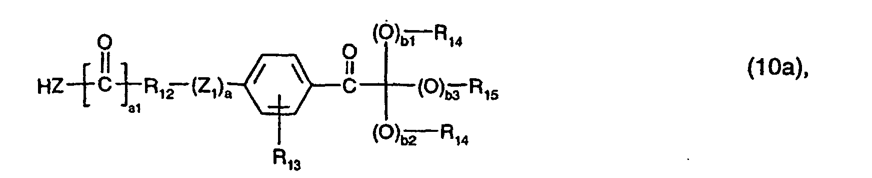 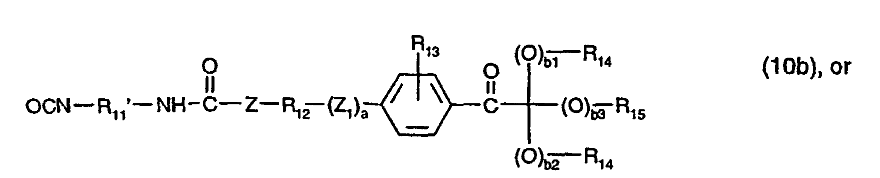 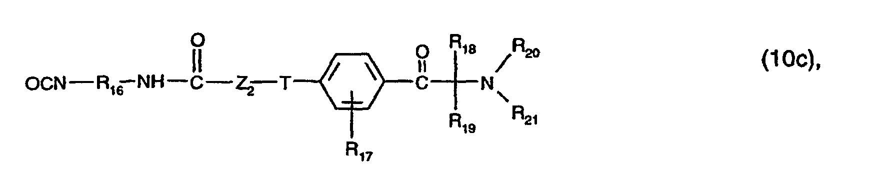 wherein Z is bivalent -O-, -NH- or -NR₂₂-; Z₁ is -O-, -O-(O)C-, -C(O)-O- or -O-C(O)-O-; R₁₃ is H, C₁-C₁₂-alkyl, C₁-C₁₂-alkoxy or N-C₁-C₁₂-alkylamino; R₁₄ and R₁₅ are each independently of the other H, linear or branched C₁-C₈-alkyl, C₁-C₈-hydroxyalkyl or C₆-C₁₀-aryl, or the groups R₁₄-(O)_{b1}- and R₁₄-(O)_{b2}- together are -(CH₂)_{c}- wherein c is an integer from 3 to 5, or the groups R₁₄-(O)_{b1}-, R₁₄-(O)_{b2}- and R₁₅-(O₁)_{b3}- together are a radical of the formula 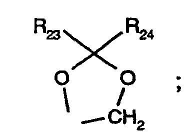 R₁₂ is a direct bond or linear or branched C₁-C₈-alkylene that is unsubstituted or substituted by -OH and/or is uninterrupted or interrupted by one or more groups -O-, -O-C(O)- or -O-C(O)-O-; R₁₁' is branched C₃-C₁₈-alkylene, unsubstituted or C₁-C₄-alkyl- or C₁-C₄-alkoxy-substituted C₆-C₁₀-arylene, or unsubstituted or C₁-C₄-alkyl- or C₁-C₄-alkoxy-substituted C₇-C₁₈-aralkylene, unsubstituted or C₁-C₄-alkyl- or C₁-C₄-alkoxy-substituted C₃-C₈-cycloalkylene, unsubstituted or C₁-C₄-alkyl- or C₁-C₄-alkoxy-substituted C₃-C₈-cycloalkylene-C_{y}H_{2y}- or unsubstituted or C₁-C₄-alkyl- or C₁-C₄-alkoxy-substituted -C_{y}H_{2y}-(C₃-C₈-cycloalkylene)-C_{y}H_{2y}- wherein y is an integer from 1 to 6; R₁₆ independently has the same definitions as R₁₁' or is linear C₃-C₁₈-alkylene; R₂₂ is linear or branched C₁-C₆-alkyl; T is bivalent -O-, -NH-, -S-, C₁-C₈-alkylene or 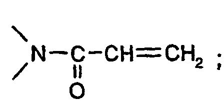 Z₂ is a direct bond or - O-(CH₂)_{d}- wherein d is an integer from 1 to 6 and the terminal CH₂ group of which is linked to the adjacent T in formula (10c); R₁₇ is H, C₁-C₁₂-alkyl, C₁-C₁₂-alkoxy, N-C₁-C₁₂-alkylamino or -NR₂₅R₂₆ wherein R₂₅ is C₁-C₈-alkyl and R₂₆ is H or C₁-C₈-alkyl; R₁₈ is linear or branched C₁-C₈-alkyl, C₂-C₈-alkenyl or C₆-C₁₀-aryl-C₁-C₈-alkyl; R₁₉ independently of R₁₈ has the same definitions as R₁₈ or is C₆-C₁₀-aryl, or R₁₈ and R₁₉ together are -(CH₂)ₑ- wherein e is an integer from 2 to 6; R₂₀ and R₂₁ are each independently of the other linear or branched C₁-C₈-alkyl that may be substituted by C₁-C₄-alkoxy, or C₆-C₁₀-aryl-C₁-C₈-alkyl or C₂-C₈-alkenyl; or R₂₀ and R₂₁ together are -(CH₂)_{f1}-Z₃-(CH₂)_{f2}- wherein Z₃ is a direct bond, -O-, -S- or -NR₂₆-, and R₂₆ is H or C₁-C₈-alkyl and f1 and f2 are each independently of the other an integer from 2 to 4; R₂₃ and R₂₄ are each independently of the other H, C₁-C₈-alkyl, C₃-C₈-cycloalkyl, benzyl or phenyl; and a, a1, b1, b2 and b3 are each independently of the other 0 or 1; subject to the provisos that b1 and b2 are each 0 when R₁₅ is H; that the total of (b1+b2+b3) is not exceeding 2; and that a is 0 when R₁₂ is a direct bond.

A preferred sub-group of compounds of formula (10a) or (10b) comprises those wherein, b1 and b2 are each 0; Z and Z₁ are each bivalent -O-; b3 is 0 or 1; R₁₄ is C₁-C₄-alkyl or phenyl, or both groups R₁₄ together are tetramethylene or pentamethylene; R₁₅ is C₁-C₄-alkyl or H, R₁₃ is hydrogen; a and a1 are each independently 0 or 1; R₁₂ is linear or branched C₂-C₄-alkylene, or is a direct bond, in which case a is 0; R₁₁' is branched C₅-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexyl-C_{y}H_{2y}- or -C_{y}H_{2y}-cyclohexyl-C_{y}H_{2y}- or cyclohexyl-C_{y}H_{2y}- or -C_{y}H_{2y}-cyclohexyl-C_{y}H_{2y}- substituted by from 1 to 3 methyl groups; y is 1 or 2; and R₁₆ has the same definitions as R₁₁' or is linear C₃-C₁₀alkylene.

An especially preferred sub-group of compounds of formula (10a) or (10b) comprises those wherein, b1 and b2 are each 0, Z and Z₁ are each bivalent -O-, b3 is 0 or 1; R₁₄ is methyl or phenyl, or both groups R₁₄ together are pentamethylene; R₁₅ is methyl or H; R₁₃ is hydrogen; a is 1 and R₁₂ is ethylene, or a is 0 and R₁₂ is a direct bond; a1 is 0 or 1; R₁₁' is branched C₆-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexyl-CH₂- or cyclohexyl-CH₂-substituted by from 1 to 3 methyl groups; R₁₆ has the same definitions as R₁₁' or is linear C₅-C₁₀alkylene.

A preferred sub-group of compounds of formula (10c) comprises those wherein T is bivalent -O-, -NH-, -S- or -(CH₂)_{y}- wherein y is an integer from 1 to 6; Z₂ is a direct bond or -O-(CH₂)_{y}- wherein y is an integer from 1 to 6 and the terminal CH₂ group of which is linked to the adjacent T in formula (1 0c); R₁₇ is H, C₁-C₁₂-alkyl or C₁-C₁₂-alkoxy; R₁₈ is linear C₁-C₈-alkyl, C₂-C₈-alkenyl or C₆-C₁₀-aryl-C₁-C₈-alkyl; R₁₉ independently of R₁₈ has the same definitions as R₁₈ or is C₆-C₁₀-aryl, or R₁₈ and R₁₉ together are -(CH₂)ₑ- wherein e is an integer from 2 to 6; R₂₀ and R₂₁ are each independently of the other linear or branched C₁-C₈-alkyl that may be substituted by C₁-C₄-alkoxy, or C₆-C₁₀-aryl-C₁-C₈-alkyl or C₂-C₈-alkenyl; or R₂₀ and R₂₁ together are -(CH₂)_{f1}-Z₃-(CH₂)_{f2}- wherein Z₃ is a direct bond, -O-, -S- or -NR₂₆-, and R₂₆ is H or C₁-C₈-alkyl and f1 and f2 are each independently of the other an integer from 2 to 4; and R₁₆ is branched C₆-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexylene-CH₂- or cyclohexylene-CH₂- substituted by from 1 to 3 methyl groups.

An especially preferred sub-group of compounds of formula (10c) comprises those wherein T is bivalent -O-; Z₂ is -O-(CH₂)_{y}- wherein y is an integer from 1 to 4 and the terminal CH₂ group of which is linked to the adjacent T in formula (10c); R₁₇ is H; R₁₈ is methyl, allyl, tolylmethyl or benzyl, R₁₉ is methyl, ethyl, benzyl or phenyl, or R₁₈ and R₁₉ together are pentamethylene, R₂₀ and R₂₁ are each independently of the other C₁-C₄-alkyl or R₂₀ and R₂₁ together are -CH₂CH₂OCH₂CH₂-, and R₁₆ is branched C₆-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexylene-CH₂- or cyclohexylene-CH₂- substituted by from 1 to 3 methyl groups.

Some examples of especially preferred functional photoinitiators are the compounds of formulae

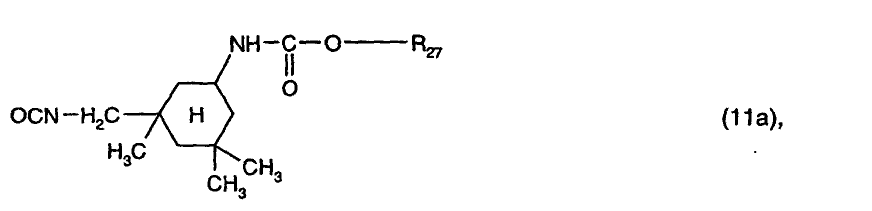

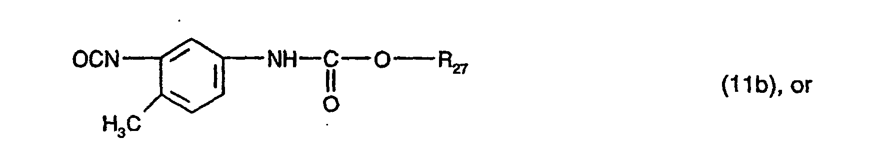

-OCN-CH₂-C(CH₃)₂-CH₂-CH(CH₃)-CH₂-CH₂-NH-C(O)-O-R₂₇ (11c),

wherein R₂₇ is a radical 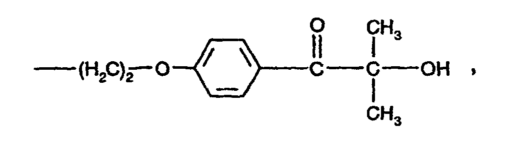 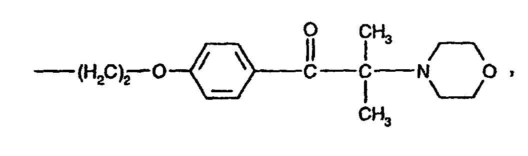 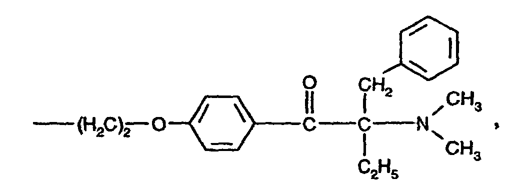 or

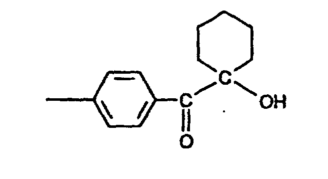

In a preferred embodiment of the invention, the covalent bonding between the bilayer(s) that is/are attached to the bulk material surface and the photoinitiator occurs via reaction of an amino or carboxy group, particularly an amino group, of the modified bulk material surface with an isocyanato group of the photoinitiator, for example using a photoinitiator of the above formula (10b), (10c), (11a), (11b) or (11c). Suitable methods for this are known, for example, from the above-mentioned documents. The reaction may be carried out, for example, at elevated temperature, for example from 0° to 100°C and preferably at room temperature, and optionally in the presence of a catalyst. After the reaction, excess compounds can be removed, for example, with solvents.

According to a preferred embodiment of the invention the bulk material comprises on its modified surface -NH₂ and/or -NH- groups, that are coreactive with isocyanato groups, some of whose H atoms have been substituted by radicals of the formulae 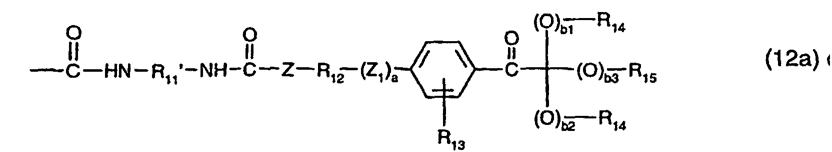 or 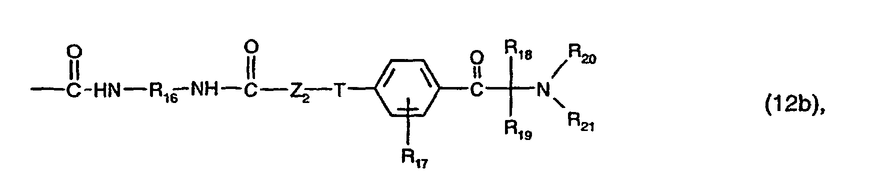 wherein for the variables R₁₁'-R₂₁, T, Z, Z₁, Z₂, a, b1, b2 and b3 the above-given meanings and preferences apply.

In another embodiment of the invention, the covalent bonding between the modified bulk material surface and the photoinitiator occurs via reaction of a carboxy or isocyanato group of the bilayer attached to the bulk material with a hydroxy, amino or alkylamino group of the photoinitiator, for example using a photoinitiator of the above formula (10a). Isocyanato groups may be attached to the bilayer, for example, by first reacting an above-mentioned modified bulk material containing a bilayer with amino groups on the surface, selectively with one isocyanato group of a diisocyanate of formula OCN-R₁₁'-NCO, wherein R₁₁' has the above-given meanings; the thus modified bulk material then may be reacted with a photoinitiator of the above-mentioned formula (10a). The reaction of carboxy groups of the bilayer with hydroxy or amino groups of the photoinitiator of formula (10a) is well-known in the art and may be carried out, for example, as described in textbooks of organic chemistry.

A hydrophilic monomer useful to provide the hydrophilic surface coating (b) on the initiator-modified bulk material surface is typical a monomer that yields as homopolymer a polymer that is water-soluble or can absorb at least 10 % by weight of water. Examples of preferred hydrophilic monomers are hydroxy-substituted C₂-C₄-alkyl acrylates and methacrylates, acrylamide, methacrylamide, N,N-di-C₁-C₄-alkyl acrylamides and methacrylamides, ethoxylated acrylates and methacrylates, hydroxy-substituted C₂-C₄-alkyl acrylamides and methacrylamides, hydroxy-substituted C₁-C₄-alkyl vinyl ethers, sodium ethylenesulfonate, sodium styrenesulfonate, 2-acrylamido-2-methylpropanesulfonic acid, N-vinylpyrrole, N-vinylsuccinimide, N-vinylpyrrolidone, 2- or 4-vinylpyridine, acrylic acid, methacrylic acid, amino-(the term "amino" also including quaternary ammonium), mono-C₁-C₄-alkylamino- or di-C₁-C₄-alkylamino-C₁-C₄-alkyl acrylates and methacrylates, allylalcohol and the like. Hydroxy-substituted or N,N-di-C₁-C₂-alkylamino-substituted C₂-C₄alkyl(meth)acrylates, five- to seven-membered N-vinyl lactams, N,N-di-C₁-C₄alkyl(meth)acrylamides and vinylically unsaturated carboxylic acids having a total of from 3 to 5 carbon atoms, for example, are preferred.

Examples of preferred hydrophilic vinylic monomers include hydroxyethyl methacrylate, hydroxyethyl acrylate, acrylamide, methacrylamide, N,N-dimethylacrylamide, allyl alcohol, N-vinylpyrrolidone, acrylic acid, methacrylic acid and N,N-dimethylaminoethyl methacrylate.

Preferably the hydrophilic surface coating (b) on the bulk material (a) is obtained using a suitable macromonomer. A preferred macromonomer is, for example, of formula 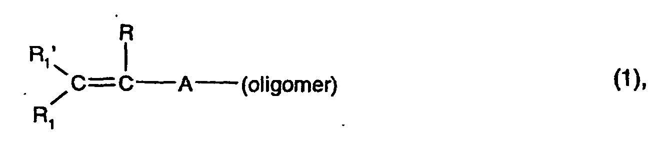 wherein
R₁ is hydrogen, C₁-C₆-alkyl or a radical -COOR';
R, R' and R₁' are each independently of the other hydrogen or C₁-C₆-alkyl;
A is a direct bond or is a radical of formula

   -C(O)-(A₁)ₙ-X- (2a)

   or

   -(A₂)ₘ-NH-C(O)-X- (2b);

   or

   -(A₂)ₘ-X-C(O)- (2c);

   or

   -C(O)-NH-C(O)-X- (2d);

   or

   -C(O)-X₁-(alk*)-X-C(O)- (2e);

   or
A and R₁, together with the adjacent double bond, are a radical of formula 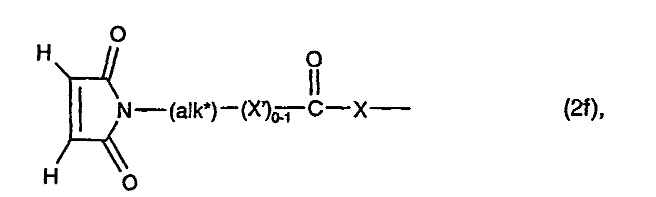
A₁ is -O-C₂-C₁₂-alkylene which is unsubstituted or substituted by hydroxy, or is -O-C₂-C₁₂-alkylene-NH-C(O)- or -O-C₂-C₁₂-alkylene-O-C(O)-NH-R₁₁-NH-C(O)-, wherein R₁₁ is linear or branched C₁-C₁₈-alkylene or unsubstituted or C₁-C₄-alkyl- or C₁-C₄-alkoxy-substituted C₆-C₁₀-arylene, C₇-C₁₈-aralkylene, C₆-C₁₀-arylene-C₁-C₂-alkylene-C₆-C₁₀-arylene, C₃-C₈-cycloalkylene, C₃-C₈-cycloalkylene-C₁-C₆-alkylene, C₃-C₈-cycloalkylene-C₁-C₂-alkylene-C₃-C₈-cycloalkylene or C₁-C₆-alkylene-C₃-C₈-cycloalkylene-C₁-C₆-alkylene ;
A₂ is C₁-C₈-alkylene; phenylene or benzylene;
m and n are each independently of the other the number 0 or 1;
X, X₁ and X' are each independently of the other a bivalent group -O- or -NR", wherein R" is hydrogen or C₁-C₆-alkyl;
(alk*) is C₂-C₁₂-alkylene;
and (oligomer) denotes
(i) the radical of a telomer of formula 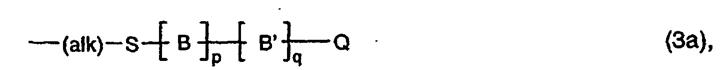 wherein
   (alk) is C₂-C₁₂-alkylene,
   Q is a monovalent group that is suitable to act as a polymerization chain-reaction terminator,
   p and q are each independently of another an integer from 0 to 250, wherein the total of (p+q) is an integer from 2 to 250,
   and B and B' are each independently of the other a 1,2-ethylene radical derivable from a copolymerizable vinyl monomer by replacing the vinylic double bond by a single bond, at least one of the radicals B and B' being substituted by a hydrophilic substituent; or
(ii) the radical of an oligomer of the formula 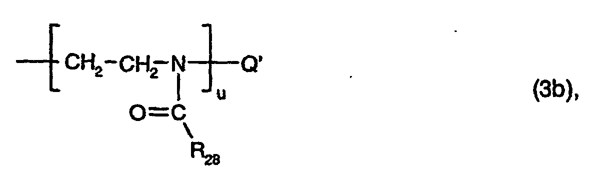 wherein R₂₈ is hydrogen or unsubstituted or hydroxy-substituted C₁-C₁₂-alkyl, u is an integer from 2 to 250 and Q' is a radical of a polymerization initiator; or
(iii) the radical of formula 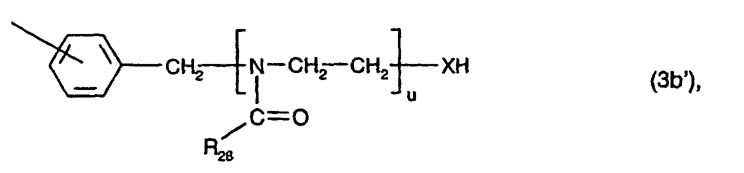 wherein R₂₈, X and u are as defined above,
(iv) the radical of an oligomer of formula 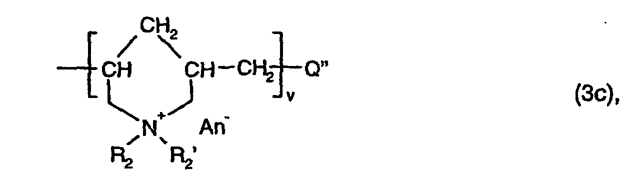 wherein R₂ and R₂' are each independently C₁-C₄-alkyl, An⁻ is an anion, v is an integer from 2 to 250, and Q" is a monovalent group that is suitable to act as a polymerization chain-reaction terminator; or
(v) the radical of an oligopeptide of formula

   -(CHR₄-C(O)-NH)ₜ-CHR₄-COOH (3d)

   or

   -CHR₄-(NH-C(O)-CHR₄)ₜ-NH₂ (3d'),

   wherein R₄ is hydrogen or C₁-C₄-alkyl which is unsubstituted or substituted by hydroxy, carboxy, carbamoyl, amino, phenyl, o-, m- or p-hydroxyphenyl, imidazolyl, indolyl or a radical -NH-C(=NH)-NH₂ and t is an integer from 2 to 250, or the radical of an oligopeptide based on proline or hydroxyproline; or
(vi) the radical of a polyalkylene oxide of formula

   -(alk**-O)_{z}-[CH₂-CH₂-O]ᵣ-[CH₂-CH(CH₃)-O]ₛ-R₃₀ (3e),

   wherein R₃₀ is hydrogen or C₁-C₂₄-alkyl, (alk**) is C₂-C₄-alkylene, z is 0 or 1, rand s are each independently an integer from 0 to 250 and the total of (r+s) is from 2 to 250; or
(vii) the radical of an oligosaccharide;
subject to the provisos that
A is not a direct bond if (oligomer) is a radical of formula (3a);
A is a direct bond if (oligomer) is a radical of formula (3b');
A is not a radical of formula (2c) or (2e) if (oligomer) is a radical of formula (3b), (3c), (3d), (3e) or is the radical of an oligosaccharide;and
A is a radical of formula (2c) or (2e) if (oligomer) is a radical of formula (3d').

The following preferences apply to the variables contained in the definition of the macromonomer of formula (1):
R' is preferably hydrogen or C₁-C₄-alkyl, more preferably hydrogen or C₁-C₂-alkyl and particularly preferably hydrogen.
R, is preferably hydrogen, methyl or carboxyl, and particularly preferably hydrogen.
R is preferably hydrogen or methyl.
X is preferably a bivalent group -O- or -NH-. X is particularly preferably the group -NH- if (oligomer) is a radical of formula (3a); (3c) or (3d), and is particularly preferably the group -O- if (oligomer) is a radical of formula (3b). X' is preferably -O- or -NH- and more preferably - NH-. X₁ is preferably -O- or -NH-.
R₁₁ as alkylene is preferably a linear or branched C₃-C₁₄alkylene radical, more preferably a linear or branched C₄-C₁₂alkylene radical and most preferably a linear or branched C₆-C₁₀-alkylene radical.

When R₁₁ is arylene, it is, for example, naphthylene or especially phenylene, each of which may be substituted, for example, by C₁-C₄-alkyl or by C₁-C₄-alkoxy. Preferably, R₁₁ as arylene is 1,3- or 1,4-phenylene that is unsubstituted or substituted by C₁-C₄-alkyl or by C₁-C₄-alkoxy in the ortho-position to at least one linkage site. Examples of substituted arylene are 1-methyl-2,4-phenylene, 1,5-dimethyl-2,4-phenylene, 1-methoxy-2,4-phenylene and 1-methyl-2,7-naphthylene.

R₁₁ as aralkylene is preferably naphthylalkylene and most preferably phenylalkylene. The alkylene group in aralkylene contains preferably from 1 to 12, more preferably from 1 to 6 and most preferably from 1 to 4 carbon atoms. Most preferably, the alkylene group in aralkylene is methylene or ethylene.

When R₁₁ is cycloalkylene, it is preferably C₅-C₆cycloalkylene and most preferably cyclohexylene that is unsubstituted or substituted by methyl.

When R₁₁ is cycloalkylene-alkylene, it is preferably cyclopentylene-C₁-C₄-alkylene and especially cyclohexylene-C₁-C₄-alkylene, each unsubstituted or mono- or poly-substituted by C₁-C₄-alkyl, especially methyl. More preferably, the group cycloalkylene-alkylene is cyclohexylene-ethylene and, most preferably, cyclohexylene-methylene, each unsubstituted or substituted in the cyclohexylene radical by from 1 to 3 methyl groups.

When R₁₁ is alkylene-cycloalkylene-alkylene, it is preferably C₁-C₄-alkylene-cyclopentylene-C₁-C₄-alkylene and especially C₁-C₄-alkylene-cyclohexylene-C₁-C₄-alkylene, each unsubstituted or mono- or poly-substituted by C₁-C₄-alkyl, especially methyl. More preferably, the group alkylene-cycloalkylene-alkylene is ethylene-cyclohexylene-ethylene and, most preferably, is methylene-cyclohexylene-methylene, each unsubstituted or substituted in the cyclohexylene radical by from 1 to 3 methyl groups.

R₁₁ as C₃-C₈-cycloalkylene-C₁-C₂-alkylene-C₃-C₈-cycloalkylene or C₆-C₁₀-arylene-C₁-C₂-alkylene-C₆-C₁₀-arylene is preferably C₅-C₆-cycloalkylene-methylene-C₅-C₆-cycloalkylene or phenylene-methylene-phenylene, each of which may be unsubstituted or substituted in the cycloalkyl or phenyl ring by one or more methyl groups.

The radical R₁₁ has a symmetrical or, preferably, an asymmetrical structure. A preferred group of radicals R₁₁ comprises those, wherein R₁₁ is linear or branched C₆-C₁₀alkylene; cyclohexylene-methylene or cyclohexylene-methylene-cyclohexylene each unsubstituted or substituted in the cyclohexyl moiety by from 1 to 3 methyl groups; or phenylene or phenylene-methylene-phenylene each unsubstituted or substituted in the phenyl moiety by methyl. The bivalent radical R₁₁ is derived preferably from a diisocyanate and most preferably from a diisocyanate selected from the group isophorone diisocyanate (IPDI), toluylene-2,4-diisocyanate (TDI), 4,4'-methylenebis(cyclohexyl isocyanate), 1,6-diisocyanato-2,2,4-trimethyl-n-hexane (TMDI), methylenebis(phenyl isocyanate), methylenebis(cyclohexyl-4-isocyanate) and hexamethylene diisocyanate (HMDI).

Preferred meanings of A₁ are unsubstituted or hydroxy-substituted -O-C₂-C₈-alkylene or a radical -O-C₂-C₆-alkylene-NH-C(O)- and particularly -O-(CH₂)₂₋₄-, -O-CH₂-CH(OH)-CH₂- or a radical -O-(CH₂)₂₋₄-NH-C(O)-. A particularly preferred meaning of A₁ is the radical -O-(CH₂)₂-NH-C(O)-.

A₂ is preferably C₁-C₆-alkylene, phenylene or benzylene, more preferably C₁-C₄-alkylene and even more preferably C₁-C₂-alkylene.
n is an integer of 0 or preferably 1. m is preferably an integer of 1.
R₁' is preferably hydrogen or methyl and particularly preferably hydrogen.
In case that (oligomer) is a radical of formula (3a), (3b), (3c), (3d), (3e) or is the radical of an oligosaccharide. A preferably denotes a radical of formula (2a) or (2b) and particularly preferably a radical of formula (2a), wherein the above given meanings and preferences apply for the variables contained therein.
A preferred group of hydrophilic macromonomers according to the invention comprises compounds of the above formula (1), wherein R is hydrogen or methyl, R₁ is hydrogen, methyl or carboxyl, R₁' is hydrogen, A is a radical of the formula (2a) or (2b) and (oligomer) is a radical of formula (3a), (3b), (3c), (3d), (3e) or is the radical of an oligosaccharide . An even more preferred group of hydrophilic macromonomers comprises compounds of the above formula (1), wherein R is hydrogen or methyl, R₁ and R₁' are each hydrogen, A is a radical of the formula (2a) and (oligomer) is a radical of formula (3a). A further group of preferred macromonomers comprises compounds of formula (1), wherein A is a radical of formula (2e) above and (oligomer) is a radical of formula (3a).

(alk) and (alk*) are each independently preferably C₂-C₈-alkylene, more preferably C₂-C₆-alkylene, even more preferably C₂-C₄-alkylene and particularly preferably 1,2-ethylene. The alkylene radicals (alk) and (alk*) may be branched or preferably linear alkylene radicals.

Q is for example hydrogen.

The total of (p+q) is preferably an integer from 2 to 150, more preferably from 5 to 100, even more preferably from 5 to 75 and particularly preferably from 10 to 50. In a preferred embodiment of the invention q is 0 and p is an integer from 2 to 250, preferably from 2 to 150, more preferably from 5 to 100, even more preferably from 5 to 75 and particularly preferably from 10 to 50. In a further preferred embodiment p is from 4 to 99, q is from 1 to 96 and the total of (p+q) is from 5 to 100.

Suitable hydrophilic substituents of the radicals B or B' may be non-ionic, anionic, cationic or zwitterionic substituents. Accordingly, the telomer chain of formula (3a) that contains monomer units B and/or B' may be a charged chain containing anionic, cationic and/or zwitterionic groups or may be an uncharged chain. In addition, the telomer chain may comprise a copolymeric mixture of uncharged and charged units. The distribution of the charges within the telomer, if present, may be random or blockwise.

In one preferrred embodiment of the invention, the telomer radical of formula (3a) is composed solely of non-ionic monomer units B and/or B'. In another preferred embodiment of the invention, the telomer radical of formula (3a) is composed solely of ionic monomer units B and/or B', for example solely of cationic monomer units or solely of anionic monomer units. Still another preferred embodiment of the invention is directed to telomer radicals of formula (3a) comprising nonionic units B and ionic units B'.

Suitable non-ionic substituents of B or B' include for example a radical C₁-C₆-alkyl which is substituted by one or more same or different substituents selected from the group consisting of -OH, C₁-C₄-alkoxy and -NR₉R₉', wherein R₉ and R₉' are each independently of another hydrogen or unsubstituted or hydroxy-substituted C₁-C₆-alkyl or phenyl; phenyl which is substituted by hydroxy, C₁-C₄-alkoxy or -NR₉R₉', wherein R₉ and R₉' are as defined above; a radical -COOY, wherein Y is C₁-C₂₄-alkyl which is unsubstituted or substituted, for example, by hydroxy, C₁-C₄-alkoxy, -O-Si(CH₃)₃, -NR₉R₉' wherein R₉ and R₉' are as defined above, a radical -O-(CH₂CH₂O)₁₋₂₄-E wherein E is hydrogen or C₁-C₆-alkyl, or a radical -NH-C(O)-O-G, wherein -O-G is the radical of a saccharide with 1 to 8 sugar units or is a radical -O-(CH₂CH₂O)₁₋₂₄-E, wherein E is as defined above, or Y is C₅-C₈-cycloalkyl which is unsubstituted or substituted by C₁-C₄-alkyl or C₁-C₄-alkoxy, or is unsubstituted or C₁-C₄-alkyl- or C₁-C₄-alkoxy-substituted phenyl or C₇-C₁₂-aralkyl; -CONY₁Y₂ wherein Y₁ and Y₂ are each independently hydrogen, C₁-C₁₂-alkyl, which is unsubstituted or substituted for example by hydroxy, C₁-C₄-alkoxy or a radical -O-(CH₂CH₂O)₁₋₂₄-E wherein E is as defined above, or Y₁ and Y₂ together with the adjacent N-atom form a five- or six-membered heterocyclic ring having no additional heteroatom or one additional oxygen or nitrogen atom; a radical -OY₃, wherein Y₃ is hydrogen; or C₁-C₁₂-alkyl which is unsubstituted or substituted by -NR₉R₉'; or is a radical -C(O)-C₁-C₄-alkyl; and wherein R₉ and R₉' are as defined above; or a five- to seven-membered heterocyclic radical having at least one N-atom and being bound in each case via said nitrogen atom.

Suitable anionic substituents of B or B' include for example C₁-C₆-alkyl which is substituted by -SO₃H, -OSO₃H, -OPO₃H₂ and -COOH; phenyl which is substituted by one or more same or different substituents selected from the group consisting of -SO₃H, -COOH, -OH and -CH₂-SO₃H; -COOH; a radical -COOY₄, wherein Y₄ is C₁-C₂₄-alkyl which is substituted for example by -COOH, -SO₃H, -OSO₃H, -OPO₃H₂ or by a radical -NH-C(O)-O-G' wherein G' is the radical of an anionic carbohydrate; a radical -CONY₅Y₆ wherein Y₅ is C₁-C₂₄-alkyl which is substituted by -COOH, -SO₃H, -OSO₃H, or -OPO₃H₂ and Y₆ independently has the meaning of Y₅ or is hydrogen or C₁-C₁₂-alkyl; or -SO₃H; or a salt thereof, for example a sodium, potassium, ammonium or the like salt thereof.

Suitable cationic substituents of B or B' include C₁-C₁₂-alkyl which is substituted by a radical -NR₉R₉'R₉"⁺An⁻, wherein R₉, R₉' and R₉" are each independently of another hydrogen or unsubstituted or hydroxy-substituted C₁-C₆-alkyl or phenyl, and An⁻ is an anion; or a radical -C(O)OY₇, wherein Y₇ is C₁-C₂₄-alkyl which is substituted by -NR₉R₉'R₉"⁺An⁻ and is further unsubstituted or substituted for example by hydroxy, wherein R₉, R₉', R₉" and An⁻ are as defined above.

Suitable zwitterionic substituents of B or B' include a radical -R₃-Zw, wherein R₃ is a direct bond or a functional group, for example a carbonyl, carbonate, amide, ester, dicarboanhydride, dicarboimide, urea or urethane group; and Zw is an aliphatic moiety comprising one anionic and one cationic group each.

The following preferences apply to the hydrophilic substituents of B and B':

### (i) non-ionic substituents:

Preferred alkyl substituents of B or B' are C₁-C₄-alkyl, in particular C₁-C₂-alkyl, which is substituted by one or more substituents selected from the group consisting of -OH and -NR₉R₉', wherein R₉ and R₉' are each independently of another hydrogen or C₁-C₄-alkyl, preferably hydrogen, methyl or ethyl and particularly preferably hydrogen or methyl, for example -CH₂-NH₂, -CH₂-N(CH₃)₂.
Preferred phenyl substituents of B or B' are phenyl which is substituted by -NH₂ or N(C₁-C₂-alkyl)₂, for example o-, m- or p-aminophenyl.
In case that the hydrophilic substituent of B or B' is a radical -COOY, Y as optionally substituted alkyl is preferably C₁-C₁₂-alkyl, more preferably C₁-C₆-alkyl, even more preferably C₁-C₄-alkyl and particularly preferably C₁-C₂-alkyl, each of which being unsubstituted or substituted as mentioned above. In case that the alkyl radical Y is substituted by -NR₉R₉', the above-given meanings and preferences apply for R₉ and R₉'.

Examples of suitable saccharide substituents -O-G of the alkyl radical Y that is substituted by -NH-C(O)-O-G are the radical of a mono- or disaccharide, for example glucose, acetyl glucose, methyl glucose, glucosamine, N-acetyl glucosamine, glucono lactone, mannose, galactose, galactosamine, N-acetyl galactosamine, fructose, maltose, lactose, fucose, saccharose or trehalose, the radical of an anhydrosaccharide such as levoglucosan, the radical of a glucosid such as octylglucosid, the radical of a sugar alcohol such as sorbitol, the radical of a sugar acid derivative such as lactobionic acid amide, or the radical of an oligosaccharide with a maximum of 8 sugar units, for example fragments of a cyclodextrin, starch, chitosan, maltotriose or maltohexaose. The radical -O-G preferably denotes the radical of a mono- or disaccharide or the radical of a cyclodextrin fragment with a maximum of 8 sugar units. Particular preferred saccharide radicals -O-G are the radical of trehalose or the radical of a cyclodextrin fragment. In case that the alkyl radical Y is substituted by a radical -O-(CH₂CH₂O)₁₋₂₄-E or -NH-C(O)-O-G wherein -O-G is -O-(CH₂CH₂O)₁₋₂₄-E, the number of (CH₂CH₂O) units is preferably from 1 to 12 in each case and more preferably from 2 to 8. E is preferably hydrogen or C₁-C₂-alkyl.
Y as C₅-C₈-cycloalkyl is for example cyclopentyl or preferably cyclohexyl, each of which being unsubstituted or substituted for example by 1 to 3 C₁-C₂-alkyl groups.Y as C₇-C₁₂-aralkyl is for example benzyl.

Preferred nonionic radicals -COOY are those wherein Y is C₁-C₄-alkyl; or C₂-C₄-alkyl which is substituted by one or two substituents selected from the group consisting of hydroxy; ; C₁-C₂-alkoxy; -O-Si(CH₃)₃; and -NR₉R₉' wherein R₉ and R₉' are each independently of another hydrogen or C₁-C₄-alkyl; or Y is a radical -CH₂CH₂-O-(CH₂CH₂O)₁₋₁₂-E wherein E is hydrogen or C₁-C₂-alkyl; or is a radical -C₂-C₄-alkylene-NH-C(O)-O-G, wherein -O-G is the radical of a saccharide.

More preferred non-ionic radicals -COOY are those wherein Y is C₁-C₂-alkyl, particularly methyl; or C₂-C₄-alkyl which is substituted by one or two substituents selected from the group consisting of -OH and -NR₉R₉' wherein R₉ and R₉' are each independently of another hydrogen or C₁-C₂-alkyl; or a radical -CH₂CH₂-O-(CH₂CH₂O)₁₋₁₂-E wherein E is hydrogen or C₁-C₂-alkyl; or is a radical -C₂-C₄-alkylene-NH-C(O)-O-G wherein -O-G is the radical of a saccharide.

Particularly preferred radicals -COOY comprise those wherein Y is C₂-C₃-alkyl, which is substituted by hydroxy or N,N-di-C₁-C₂-alkylamino, or is a radical -C₂-C₃-alkylene-NH-C(O)-O-G wherein -O-G is the radical of trehalose.

Preferred non-ionic substituents -C(O)-NY₁Y₂ of B or B' are those wherein Y₁ and Y₂ are each independently of the other hydrogen or C₁-C₄-alkyl which is unsubstituted or substituted by hydroxy; or Y₁ and Y₂ together with the adjacent N-atom form a heterocyclic 6-membered ring having no further heteroatom or having one further N- or O-atom. Even more preferred meanings of Y₁ and Y₂, independently of each other, are hydrogen or C₁-C₂-alkyl which is unsubstituted or substituted by hydroxy; or Y₁ and Y₂ together with the adjacent N-atom form a N-C₁-C₂-alkylpiperazino or morpholino ring. Particularly preferred non-ionic radicals -C(O)-NY₁Y₂ are those wherein Y₁ and Y₂ are each independently of the other hydrogen or C₁-C₂-alkyl; or Y₁ and Y₂ together with the adjacent N-atom form a morpholino ring.

Preferred non-ionic substituents -OY₃ of B or B' are those wherein Y₃ is hydrogen, C₁-C₄-alkyl which is unsubstituted or substituted by -NH₂ or -N(C₁-C₂-alkyl)₂, or is a group -C(O)C₁-C₂-alkyl, Y₃ is particularly preferred hydrogen or acetyl.

Preferred non-ionic heterocyclic substituents of B or B' are a 5- or 6-membered heteroaromatic or heteroaliphatic radical having one N-atom and in addition no further heteroatom or an additional N- or O- heteroatom, or is a 5 to 7-membered lactame. Examples of such heterocyclic radicals are N-pyrrolidonyl, 2- or 4-pyridinyl, 2-methyl pyridin-5-yl, 2-, 3- oder 4-hydroxypyridinyl, N-ε-caprolactamyl, N-imidazolyl, 2-methylimidazol-1-yl, N-morpholinyl or 4-N-methylpiperazin-1-yl, particularly N-morpholinyl or N-pyrrolidonyl.

A group of preferred non-ionic substituents of B or B' comprises C₁-C₂-alkyl, which is unsubstituted or substituted by -OH or -NR₉R₉', wherein R₉ and R₉' are each independently of the other hydrogen or C₁-C₂-alkyl; a radical -COOY wherein Y is C₁-C₄-alkyl; C₂-C₄-alkyl which is substituted by -OH or -NR₉R₉' wherein R₉ and R₉' are each independently of another hydrogen or C₁-C₂-alkyl, or Y is a radical -C₂-C₄-alkylene-NH-C(O)-O-G wherein -O-G is the radical of a saccharide; a radical -C(O)-NY₁Y₂, wherein Y₁ and Y₂ are each independently of the other hydrogen or C₁-C₄-alkyl which is unsubstituted or substituted by hydroxy, or Y₁ and Y₂ together with the adjacent N-atom form a heterocyclic 6-membered ring having no further heteroatom or having one further N- or O-atom; a radical -OY₃, wherein Y₃ is hydrogen, C₁-C₄-alkyl which is unsubstituted or substituted by -NH₂ or -N(C₁-C₂-alkyl)₂, or is a group -C(O)C₁-C₂-alkyl; or a 5- or 6-membered heteroaromatic or heteroaliphatic radical having one N-atom and in addition no further heteroatom or an additional N-, O- or S-heteroatom, or a 5 to 7-membered lactame.

A group of more preferred non-ionic substituents of B or B' comprises a radical -COOY, wherein Y is C₁-C₂-alkyl, C₂-C₃-alkyl, which is substituted by hydroxy, amino or N,N-di-C₁-C₂-alkylamino, or is a radical -C₂-C₄-alkylene-NH-C(O)-O-G wherein -O-G is the radical of trehalose; a radical -CO-NY₁Y₂, wherein Y₁ and Y₂ are each independently of the other hydrogen or C₁-C₂-alkyl which is unsubstituted or substituted by hydroxy, or Y₁ and Y₂ together with the adjacent N-atom form a N-C₁-C₂-alkylpiperazino or morpholino ring; or a heterocyclic radical selected from the group consisting of N-pyrrolidonyl, 2- or 4-pyridinyl, 2-methylpyridin-5-yl, 2-, 3- oder 4-hydroxypyridinyl, N-ε-caprolactamyl, N-imidazolyl, 2-methylimidazol-1-yl, N-morpholinyl and 4-N-methylpiperazin-1-yl.

A particularly preferred group of non-ionic substituents of B or B' comprises the radicals -CONH₂, -CON(CH₃)₂, -CONH-(CH₂)₂-OH, 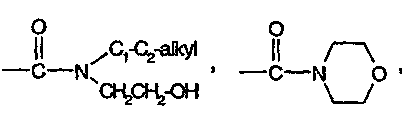 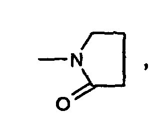 -COO-(CH₂)₂-N(CH₃)₂, and -COO(CH₂)₂₋₄-NHC(O)-O-G wherein -O-G is the radical of trehalose.

### (ii) anionic substituents:

Preferred anionic substituents of B or B' are C₁-C₄-alkyl, in particular C₁-C₂-alkyl, which is substituted by one or more substituents selected from the group consisting of -SO₃H and -OPO₃H₂, for example -CH₂-SO₃H; phenyl which is substituted by -SO₃H or sulfomethyl, for example o-, m- or p-sulfophenyl or o-, m- or p-sulfomethylphenyl; -COOH; a radical -COOY₄, wherein Y₄ is C₂-C₆-alkyl which is substituted by -COOH, -SO₃H, -OSO₃H, -OPO₃H₂, or by a radical -NH-C(O)-O-G' wherein G' is the radical of lactobionic acid, hyaluronic acid or sialic acid, in particular C₂-C₄-alkyl which is substituted by -SO₃H or -OSO₃H; a radical -CONY₅Y₆ wherein Y₅ is C₁-C₆-alkyl substituted by sulfo, in particular C₂-C₄-alkyl substituted by sulfo, and Y₆ is hydrogen, for example the radical -C(O)-NH-C(CH₃)₂-CH₂-SO₃H; or -SO₃H; or a suitable salt thereof. Particular preferred anionic substituents of B or B' are -COOH, -SO₃H, o-, m- or p-sulfophenyl, o-, m- or p-sulfomethylphenyl or a radical -CONY₅Y₆ wherein Y₅ is C₂-C₄-alkyl substituted by sulfo, and Y₆ is hydrogen, especially carboxy.

### (iii) cationic substituents:

Preferred cationic substituents of B or B' are C₁-C₄-alkyl, in particular C₁-C₂-alkyl, which is in each case substituted by -NR₉R₉'R₉"⁺An⁻; or a radical -C(O)OY₇ wherein Y₇ is C₂-C₆-alkyl, in particular C₂-C₄-alkyl, which is in each case substituted by -NR₉R₉'R₉"⁺An⁻ and is further unsubstituted or substituted by hydroxy. R₉, R₉' and R₉" are each independently of another preferably hydrogen or C₁-C₄-alkyl, more preferably methyl or ethyl and particularly preferably methyl. Examples of suitable anions An⁻ are Hal⁻, wherein Hal is halogen, for example Br⁻, F, J⁻ or particularly Cl⁻, furthermore HCO₃⁻, CO₃²⁻, H₂PO₃⁻, HPO₃²⁻, PO₃³⁻, HSO₄⁻, SO₄²⁻ or the radical of an organic acid such as OCOCH₃⁻ and the like. A particularly preferred cationic substituent of B or B' is a radical -C(O)OY₇ wherein Y₇ is C₂-C₄-alkyl, which is substituted by -N(C₁-C₂-alkyl)₃⁺An⁻ and is further substituted by hydroxy, and An⁻ is an anion, for example the radical -C(O)O-CH₂-CH(OH)-CH₂-N(CH₃)₃⁺An⁻.

### (iv) zwitterionic substituents -R₃-Zw:

R₃ is a preferably a carbonyl, ester or amide functional group and more preferably an ester group -C(O)-O-.
Suitable anionic groups of the moiety Zw are for example -COO⁻, -SO₃⁻, -OSO₃⁻, -OPO₃H⁻ or bivalent -O-PO₂⁻- or -O-PO₂⁻-O-, preferably a group -COO⁻ or -SO₃⁻ or a bivalent group - O-PO₂⁻-, and in particular a group -SO₃⁻.
Suitable cationic groups of the moiety Zw are for example a group -NR₉R₉'R₉"⁺ or a bivalent group -NR₉R₉'⁺-, wherein R₉, R₉' and R₉" are as defined above, and are each independently of the other, preferably hydrogen or C₁-C₆-alkyl, preferably hydrogen or C₁-C₄-alkyl and most preferably each methyl or ethyl.

The moiety Zw is for example C₂-C₃₀-alkyl, preferably C₂-C₁₂-alkyl, and more preferably C₃-C₈-alkyl, which is in each case uninterrupted or interrupted by -O- and substituted or interrupted by one of the above-mentioned anionic and cationic groups each, and, in addition, is further unsubstituted or substituted by a radical -OY₈, wherein Y₈ is hydrogen or the acyl radical of a carboxylic acid.

Y₈ is preferably hydrogen or the acyl radical of a higher fatty acid.

Zw is preferably C₂-C₁₂-alkyl and even more preferably C₃-C₈-alkyl which is substituted or interrupted by one of the above-mentioned anionic and cationic groups each, and in addition may be further substituted by a radical -OY₈.

A preferred group of zwitter-ionic substituents -R₃-Z corresponds to the formula

-C(O)O-(alk''')-N(R₉)₂⁺-(alk')-An⁻

or

-C(O)O-(alk")-O-PO₂⁻-(O)₀₋₁-(alk''')-N(R₉)₃⁺

wherein R₉ is hydrogen or C₁-C₆-alkyl; An⁻ is an anionic group -COO⁻, -SO₃⁻, -OSO₃⁻ or - OPO₃H⁻, preferably -COO⁻ or -SO₃⁻ and most preferably -SO₃⁻, alk' is C₁-C₁₂-alkylene, (alk") is C₂-C₂₄-alkylene which is unsubstituted or substituted by a radical -OY₈, Y₈ is hydrogen or the acyl radical of a carboxylic acid, and (alk''') is C₂-C₈-alkylene.

(alk') is preferably C₂-C₈-alkylene, more preferably C₂-C₆-alkylene and most preferably C₂-C₄-alkylene. (alk") is preferably C₂-C₁₂-alkylene, more preferably C₂-C₆-alkylene and particularly preferably C₂-C₃-alkylene which is in each case unsubstituted or substituted by hydroxy or by a radical -OY₈. (alk''') is preferably C₂-C₄-alkylene and more preferably C₂-C₃-alkylene. R₉ is hydrogen or C₁-C₄-alkyl, more preferably methyl or ethyl and particularly preferably methyl. A preferred zwitterionic substituent of B or B' is of formula

-C(O)O-CH₂-CH(OY₈)-CH₂-O-PO₂⁻-(CH₂)₂-N(CH₃)₃⁺,

wherein Y₈ is hydrogen or the acyl radical of a higher fatty acid.

In one embodiment of the invention one of B and B' may also be the radical of a hydrophobic comonomer which includes especially those customarily used in the manufacture of contact lenses. Suitable hydrophobic vinylic comonomers include, without the list being exhaustive acrylonitrile, methacrylonitrile, vinyl-C₁-C₁₈-alkanoates, C₂-C₁₈-alkenes, C₂-C₁₈-haloalkenes, styrene, C₁-C₆-alkylstyrene, C₂-C₁₀-perfluoroalkyl acrylates and methacrylates or correspondingly partially fluorinated acrylates and methacrylates, C₃-C₁₂-perfluoroalkyl-ethyl-thiocarbonylaminoethyl acrylates and methacrylates, acryloxy- and methacryloxy-alkylsiloxanes, N-vinylcarbazole and the like. Examples of suitable hydrophobic vinylic comonomers include acrylonitrile, methacrylonitrile, vinyl acetate, vinyl propionate, vinylbutyrate, vinyl valerate, styrene, chloroprene, vinyl chloride, vinylidene chloride, 1-butene, butadiene, vinyltoluene, perfluorohexylethylthiocarbonylaminoethyl methacrylate, trifluoroethyl methacrylate, hexafluoroisopropyl methacrylate, hexafluorobutyl methacrylate, tris-trimethylsilyloxy-silyl-propyl methacrylate, 3-methacryloxypropylpentamethyldisiloxane and bis(methacryloxypropyl)tetramethyldisiloxane.

B denotes for example a radical of formula 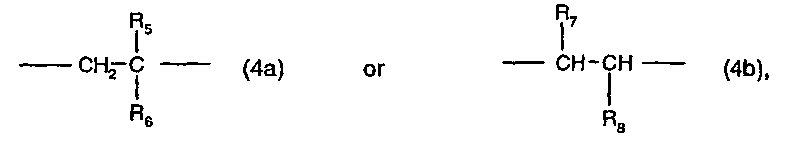 wherein R₅ is hydrogen or C₁-C₄-alkyl, preferably hydrogen or methyl; R₆ is a hydrophilic substituent, wherein the above given meanings and preferences apply; R₇ is C₁-C₄-alkyl, phenyl or a radical -C(O)OY₉, wherein Y₉ is hydrogen or unsubstituted or hydroxy-substituted C₁-C₄-alkyl; and R₈ is a radical -C(O)Y₉' or -CH₂-C(O)OY₉' wherein Y₉' independently has the meaning of Y₉.

R₇ is preferably C₁-C₂-alkyl, phenyl or a group -C(O)OY₉. R₈ is preferably a group -C(O)OY₉' or -CH₂-C(O)OY₉' wherein Y₉ and Y₉' are each independently of the other hydrogen, C₁-C₂-alkyl or hydroxy-C₁-C₂-alkyl. Particularly preferred -CHR₇-CHR₈- units according to the invention are those wherein R₇ is methyl or a group -C(O)OY₉ and R₈ is a group -C(O)OY₉' or -CH₂-C(O)OY₉' wherein Y₉ and Y₉' are each hydrogen, C₁-C₂-alkyl or hydroxy-C₁-C₂-alkyl.

B' independently may have one of the meanings given above for B or is the radical of a hydrophobic comonomer, for example the radical of one of the above-given hydrophobic comonomers.

If ( oligomer) is a telomer radical of formula (3a), the radical -(alk)-S-[B]ₚ-[B']_{q}-Q preferably denotes a radical of formula 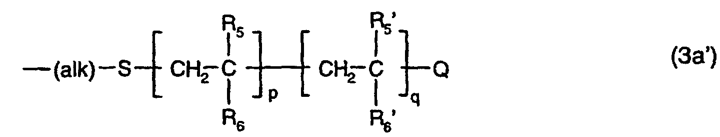 and even more preferably of the formula 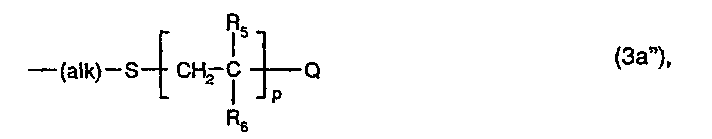 wherein for R₅, R₆, Q, p and q the above-given meanings and preferences apply, for R₅' independently the meanings and preferences given before for R₅ apply, and for R₆' independently the meanings and preferences given before for R₆ apply or R₆' is a hydrophobic substituent selected from the group consisting of hydrogen, -CN, C₁-C₁₈-alkanoyl, C₁-C₁₆-alkyl, C₁-C₁₆-haloalkyl, phenyl, C₁-C₆-alkylphenyl, C₂-C₁₀-perfluoroalkyloxycarbonyl or a corresponding partially fluorinated alkyloxycarbonyl radical, C₃-C₁₂-perfluoroalkyl-ethyl-thiocarbonylaminoethyloxycarbonyl, alkylsiloxyloxycarbonyl and carbazolyl

A preferred group of suitable hydrophilic macromers according to the invention comprises compounds of the above formula (1) wherein R is hydrogen or methyl, R₁ is hydrogen, methyl or carboxyl, R₁' is hydrogen, A is a radical of the above formula (2a), (2b) or (2e), wherein n and m are each 0 or 1, X and X₁ are each independently of the other -O- or -NH-, A₁ is unsubstituted or hydroxy-substituted -O-C₂-C₈-alkylene or a radical -O-C₂-C₆-alkylene-NH-C(O)-, A₂ is C₁-C₄-alkylene, phenylene or benzylene, (alk*) is C₂-C₄-alkylene, and (oligomer) denotes a radical of formula 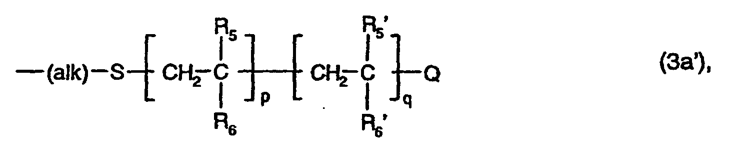 wherein (alk) is C₂-C₆-alkylene, Q is a monovalent group that is suitable to act as a polymerization chain-reaction terminator, p and q are each an integer of from 0 to 100 and the total of (p+q) is from 5 to 100, R₅ and A₅' are each independently of the other hydrogen or methyl, and for R₆ and A₆' each independently of the other the meanings and preferences given before apply. One particularly preferred embodiment of the above outlined hydrophilic macromers comprises those wherein q is 0, p is from 5 to 100, R₅ is hydrogen or methyl, and R₆ is a radical -CONH₂, -CON(CH₃)₂, -CONH-(CH₂)₂-OH, 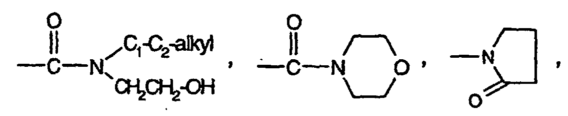 -COO-(CH₂)₂-N(CH₃)₂, or -COO(CH₂)₂₋₄-NHC(O)-O-G wherein -O-G is the radical of trehalose. A further preferred embodiment of the above outlined hydrophilic macromers comprises those wherein p is from 4 to 99, q is from 1 to 96 wherein in the total of (p+q) is from 5 to 100, R₅ and R₅' are each independently hydrogen or methyl, R₆ is a radical -CONH₂, -CON(CH₃)₂, -CONH-(CH₂)₂-OH, 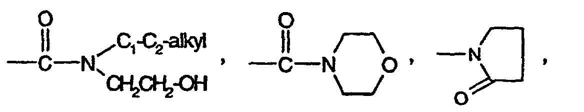 -COO-(CH₂)₂-N(CH₃)₂, or -COO(CH₂)₂₋₄-NHC(O)-O-G wherein -O-G is the radical of trehalose, and R₆' independently has the meaning of R₆ or is carboxy, subject to the proviso that R₆ and R₆' are different.

A more preferred group of suitable hydrophilic macromonomers according to the invention comprises compounds of formula 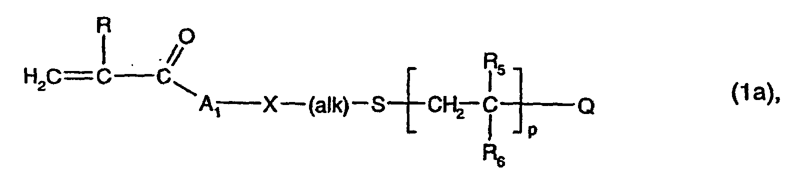 wherein R is hydrogen or methyl, A₁ is -O-(CH₂)₂₋₄-, -O-CH₂-CH(OH)-CH₂- or a radical -O-(CH₂)₂₋₄-NH-C(O)-, X is -O- or -NH-, (alk) is C₂-C₄-alkylene, Q is a monovalent group that is suitable to act as a polymerization chain-reaction terminator, p is an integer from 5 to 50, R₅ is hydrogen or methyl, and for R₆ the above given meanings and preferences apply.

A particularly preferred embodiment of the invention relates to hydrophilic macromonomers of the formula 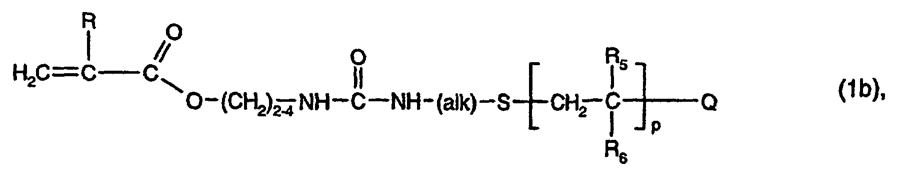 wherein for R, R₅, R₆, Q, (alk) and p the above-given meanings and preferences apply. A particularly preferred group of hydrophilic macromonomers are compounds of the above formula (1b) wherein R is hydrogen or methyl, (alk) is C₂-C₄-alkylene, R₅ is hydrogen or methyl, p is an integer of 5 to 50, Q is as defined before, and for R₆ the above given meanings and preferences apply.

If (oligomer) is a radical (ii) of formula (3b), Q' in formula (3b) is for example C₁-C₁₂-alkyl, phenyl or benzyl, preferably C₁-C₂-alkyl or benzyl and in particular methyl. R₂₉ is preferably unsubstituted or hydroxy-substituted C₁-C₄-alkyl and in particular methyl. u is preferably an integer from 2 to 150, more preferably from 5 to 100, even more preferably from 5 to 75 and particularly preferably from 10 to 50.

If (oligomer) is a radical of formula (3b'), the above given meanings and preferences apply for the variables X, R₂₉ and u contained therein.

If (oligomer) denotes a radical (iv) of formula (3c),R₂ and R₂' are each preferably ethyl or in particular methyl; v is preferably an integer from 2 to 150, more preferably from 5 to 100, even more preferably from 5 to 75 and particularly preferably from 10 to 50; Q" is for example hydrogen; and An⁻ is as defined before.

If (oligomer) denotes an oligopeptide radical (v) of formula (3d) or 3d'), R₄ is for example hydrogen, methyl, hydroxymethyl, carboxymethyl, 1-hydroxyethyl, 2-carboxyethyl, isopropyl, n-, sec. or iso-butyl, 4-amino-n-butyl, benzyl, p-hydroxybenzyl, imidazolylmethyl, indolylmethyl or a radical -(CH₂)₃-NH-C(=NH)-NH₂. t is preferably an integer from 2 to 150, more preferably from 5 to 100, even more preferably from 5 to 75 and particularly preferably from 10 to 50.

If (oligomer) denotes a polyoxyalkylene radical (vi) of formula (3e), R₃₀ is preferably hydrogen or C₁-C₁₈-alkyl, more preferably hydrogen or C₁-C₁₂-alkyl, even more preferably hydrogen, methyl or ethyl, and particularly preferably hydrogen or methyl. (alk**) is preferably a C₂-C₃-alkylene radical. z is preferably 0. r and s are each independently preferably an integer from 0 to 100 wherein the total of (r+s) is 5 to 100. r and s are each independently more preferably an integer from 0 to 50 wherein the total of (r+s) is 8 to 50.

In a particularly preferred embodiment of the polyoxyalkylene radicals (oligomer), r is an integer from 8 to 50 and particularly 9 to 25, and s is 0.

(oligomer) as the radical of an oligosaccharide (vii) may be, for example, a di- or polysaccharide including carbohydrate containing fragments from a biopolymer. Examples are the radical of a cyclodextrin, trehalose, cellobiose, maltotriose, maltohexaose, chitohexaose or a starch, hyaluronic acid, deacetylated hyaluronic acid, chitosan, agarose, chitin 50, amylose, glucan, heparin, xylan, pectin, galactan, glycosaminoglycan, mucin, dextran, aminated dextran, cellulose, hydroxyalkylcellulose or carboxyalkylcellulose oligomer, each of which with a molecular weight average weight of, for example, up to 25000, preferably up to 10000. Preferably the oligosaccharide according to (vii) is the radical of a cyclodextrin with a maximum of 8 sugar units.

In the above formulae (2a), (2b), (2c), (2d) and (2e), the left bond is in each case attached to the double bond whereas the right bond is linked to the oligomer. Formulae (3a), (3a') and (3e) are to be understood as a statistic description of the respective oligomeric radicals, that is to say, the orientation of the monomers and the sequence of the monomers (in case of copolymers) are not fixed in any way by said formulae. The arrangement of B and B' in formula (3a) or of the ethyleneoxide and propyleneoxide units in formula (3e) thus may be random or blockwise. Throughout the whole description, anions such as -COOH or -SO₃H groups always include suitable salt forms, preferably biomedical or especially ophthalmically acceptable salts, in particular -COO⁻Ka⁺ and -SO₃⁻Ka⁺ groups wherein Ka⁺ is a cation such as an alkali metal cation or an ammonium cation.

The weight average molecular weight of the macromonomers of the invention depends principally on the desired properties and is for example from 300 to 50000, preferably from 300 to 12000, more preferably from 300 to 8000, even more preferably 300 to 5000, and particularly preferably from 500 to 2000.

The macromonomers of formula (1) may be prepared by methods, for example as described in WO 99/57581.

The hydrophilic monomers and macromonomers may be applied to the initiator-modified bulk material surface and polymerized there according to processes known per se. For example, the bulk material is immersed in a solution of the monomer or macromonomer, or a layer of monomer or macromonomer is first of all deposited on the modified bulk material surface, for example, by dipping, spraying, spreading, knife coating, pouring, rolling, spin coating or vacuum vapor deposition. The polymerization of the macromonomer on the bulk material surface then may be initiated, for example, thermally by the action of heat or preferably by irradiation, particularly by UV radiation. Suitable light sources for the irradiation are known to the artisan and comprise for example mercury lamps, high pressure mercury lamps, xenon lamps, carbon arc lamps or sunlight. The time period of irradiation may depend for example on the desired properties of the resulting composite material but is usually in the range of up to 30 minutes, preferably from 10 secondes to 10 minutes, and particularly preferably from 0.5 to 5 minutes. The irradiation may be carried out under ambient conditions or in an atmosphere of an inert gas, for example nitrogen. After the polymerization, any non-covalently bonded polymers, oligomers or non-reacted monomer or macromonomers formed can be removed, for example by treatment with suitable solvents.

By means of the above-described coating process, hydrophilic monomers may be grafted to the bulk material surface with formation of a coating having for example a so-called brush-type structure.

Most important, the grafting of the macromonomers to the bulk material surface yields a coating having for example a so-called bottle brush-type structure (BBT) composed of tethered "hairy" chains. Such BBT structures in one embodiment comprise a long hydrophilic or hydrophobic backbone which carries relatively densely packed comparatively short hydrophilic side chains (called primary bottle brushes). Another embodiment relates to secondary bottle brushes which are characterized in that the hydrophilic side chains themselves carry densely packed hydrophilic "secondary" side chains. Polymeric coatings of said primary and secondary BBT structures to a certain extent mimic highly water-retaining structures occurring in the human body, for example in cartilage or mucosal tissue.

The coating thickness of the hydrophilic surface coating (b) depends principally on the desired properties. In case of macromonomers it can be used, for example, from 0.001 to 1000 µm, preferably from 0.01 to 500 µm, more preferably from 0.01 to 100 µm, even more preferably from 0.05 to 50 µm, especially preferably from 0.1 to 5 µm and particularly preferably from 0.1 to 1 µm. A particularly suitable range is from 0.2 to 0.6 µm.

The complete coating of the bulk material according to the invention consists (a) of a polyionic material comprising one polyelectrolyte or preferably one or more bilayers of polyelectrolytes and (b) of an upper hydrophilic coating obtainable by grafting one or more hydrophilic monomers or preferably macromonomers onto the surface, wherein the latter makes up at least 50 %, preferably from 75 to 98 % and particularly preferably from 80 to 95 % of the total thickness of the fully hydrated coating.

A further embodiment of the invention is a biomedical device, e.g. an ophthalmic device, preferably a contact lens including both hard and particularly soft contact lenses, an intraocular lens or artificial cornea, comprising a composite material according to the invention and particular a composite material comprising a macromonomer-based surface coating. The inventive materials are further useful for example as wound healing dressings, eye bandages, materials for the sustained release of an active compound such as a drug delivery patch, moldings that can be used in surgery, such as heart valves, vascular grafts, catheters, artificial organs, encapsulated biologic implants, e.g. pancreatic islets, materials for prostheses such as bone substitutes, or moldings for diagnostics, membranes or biomedical instruments or apparatus.

The biomedical devices, e.g. ophthalmic devices according to the invention have a variety of unexpected advantages over those of the prior art which make those devices very suitable for practical purposes,e.g. as contact lens for extended wear or intraocular lens. For example, they do have a high surface wettability which can be demonstrated by their contact angles, their water retention ability and their water-film break up time or tear film break up time (TBUT).

The TBUT plays an particularly important role in the field of ophthalmic devices such as contact lenses. Thus the facile movement of an eyelid over a contact lens has proven important for the comfort of the wearer; this sliding motion is facilitated by the presence of a continuous layer of tear fluid on the contact lens, a layer which lubricates the tissue/lens interface. However, clinical tests have shown that currently available contact lenses partially dry out between blinks, thus increasing friction between eyelid and the lens. The increased friction results in soreness of the eyes and reduced movement of the contact lenses. Taking into account the average time period between two blinks of an eye it follows that a wettable and biocompatible contact lens should hold a continuous layer of tear fluid for more than 10 seconds and preferably for more than 15 seconds. Whereas current biomedical materials in general have TBUTs of well below 10 seconds and thus do not reach this target, the composite materials of the present invention have TBUTs of >10 seconds and especially > 15 seconds. In addition, the TBUT of commercial contact lenses may be improved considerably by applying a surface coating according to the invention. For example, the TBUT of commercial contact lenses such as Focus Dailies™, Focus New Vues® or Lotrafilcon A lenses, may be increased by more than 50 % or, according to a particularly preferred embodiment, by ≥100 % by applying a surface coating according to the invention. On the base curve of a contact lens, the pronounced lubricity of the coating facilitates the on-eye lens movement which is essential for extended wear of contact lenses. Moreover, the composite materials of the invention provide additional effects being essential for lenses for extended wear, such as an increased thickness of the pre-lens tear film and of the topical lipid layer of the tear film which each contributes substantially to low microbial adhesion and resistance to deposit formation. Due to the extremely soft and lubricious character of the novel surface coatings, biomedical articles such as in particular contact lenses made from an inventive composite material show a superior wearing comfort including improvements with respect to late day dryness, long term (overnight) wear and acute vision on awake. The novel surface coatings moreover interact in a reversible manner with occular mucus which contributes to the improved wearing comfort.

In addition, biomedical devices, e.g. ophthalmic devices such as contact lenses, comprising a composite material of the invention have a very pronounced biocompatibility combined with good mechanical properties. For example, the devices are blood compatible and have a good tissue integration. In addition, there are generally no adverse eye effects observed, while the adsorption of proteins or lipids is low, also the salt deposit formation is lower than with conventional contact lenses. Generally, there is low fouling, low microbial adhesion and low bioerosion while good mechanical properties can be for example found in a low friction coefficient and low abrasion properties. Moreover, the dimensional stability of the composite materials of the invention is excellent. In addition, the attachment of a hydrophilic surface coating at a given bulk material according to the invention does not affect its visual transparency.

In summary, the ophthalmic devices according to the invention, such as intraocular lenses and artificial cornea or particularly contact lenses, provide a combination of low spoilation with respect to cell debris, cosmetics, tear components, lipids, proteins, salts, dust or dirt, solvent vapors or chemicals, with a high comfort for the patient wearing such opthalmic devices in view of the soft hydrogel surface which for example provides a very good on-eye movement of the ohthalmic device.

Biomedical devices such as renal dialysis membranes, blood storage bags, pacemaker leads or vascular grafts made of the composite materials of the invention resist fouling by proteins by virtue of the continuous layer of bound water, thus reducing the rate and extent of thrombosis. Blood-contacting devices fabricated according to the present invention are therefore haemocompatible and biocompatible.

In the examples, if not indicated otherwise, amounts are amounts by weight, temperatures are given in degrees Celsius. Tear break-up time values in general relate to the pre-lens tear film non-invasive break-up time (PLTF-NIBUT) that is determined following the procedure published by M. Guillon et al., Ophthal. Physiol. Opt. 9, 355-359 (1989) or M. Guillon et al., Optometry and Vision Science 74, 273-279 (1997). Average advancing and receding water contact angles of coated and non-coated lenses are determined with the dynamic Wilhelmy method using a Krüss K-12 instrument (Krüss GmbH, Hamburg, Germany). Wetting force on the solid is measured as the solid is immersed in or withdrawn from a liquid of known surface tension.

### Example A-1 (Preparation of aminofunctionalized contact lenses by attaching a bilayer)

a.) A 0.001 M polyacrylic acid (PAA) solution (Mₙ≈ 68000) is prepared by adding 0.29 grams of a 25% aqueous PAA stock solution to 1000ml of ultra-pure water in a beaker. Then the pH of the solution is adjusted to 2.5 by adding 1N HCl and the solution is filtered using qualitative filter paper.
b.) A 0.001 M polyallylamine hydrochloride (PAH) solution (Mₙ≈ 70000) is prepared by adding 0.09 g PAH (solid) into a small beaker; dissolving in ultra-pure (UP) water and transfering into a bigger beaker with a final volume of 1000 ml aqueous solution. The pH is then adjusted to 4.5 as measured with a pH meter. The solution is then filtered using qualitative filter paper.
c.) Swollen non-coated Lotrafilcon A lenses (polysiloxane/perfluoroalkylpolyether copolymer) in iso-propanol (IPA) are individually immersed into the solution a.) for 5 minutes. After this time, the lenses are withdrawn from the solution a.) and directly immersed into the solution b.) for additional 5 minutes. No water rinse is done between these two dips. After this, the lenses are released into UP water and stored at 4°C for further use.

### Example A-2 (Preparation of aminofunctionalized contact lenses by attachment of a bilayer)

a.) A 0.1 % by weight solution of a branched polyacrylic acid (Carbopol® 981 NF) is prepared by adding 0.05 g of Carbopol® 981 NF (BFGoodrich) to 50 ml of isopropanol-ultra-pure water mixture (1:4) in a beaker. After complete dissolution (overnight), the pH of the solution is adjusted to 2.5 by adding 1N HCl and the solution is filtered using qualitative filter paper.
b.) 100 ml of 0.05 % solution of polyethyleneimine (PEI) is prepared by adding 0.1 g of 50 % aqueous PEI stock solution into a mixture of isopropanol-ultra-pure water 1:4. The pH is then adjusted to 3.5 by adding 1N HCl as measured by pH meter. The solution is then filtered using qualitative filter paper.
c.) Swollen non-coated Lotrafilcon A lenses in iso-propanol (IPA) are individually immersed into the solution a.) for 10 minutes. The lenses are withdrawn from the solution a.) rinsed with ultra-pure water and immersed into the solution b.) for additional 10 minutes. After this, the lenses are released into ultra-pure water and stored at 4°C for further use.

### Example B-1 (Surface binding of reactive photoinitiator molecules)

The aminofunctionalized contact lenses from Example A-1 are first immersed into acetonitrile for 1 hour (20 ml acetonitrile / lens). The lenses are then withdrawn and directly immersed into a 1% by weight solution of the reactive photoinitiator prepared by the addition reaction from isophorone diisocyanate and 4-(2-hydroxyethoxy)phenyl 2-hydroxy-2-propyl ketone (Darocure 2959) (synthesis see EP 0 632 329) in acetonitrile. 3 drops of triethylamine (TEA) are then added to the solution. The amino groups on the lens surface react with the isocyanato groups of the photoinitiator molecules for 12 hours. After this time, the lenses are withdrawn from the reaction solution, 3x washed and extracted in acetonitrile for 8 hours and dried under reduced pressure for 2 hours. The dried lenses are subsequently used for photografting.

### Example B-2 (Surface binding of the reactive photoinitiator molecules)

The aminofunctionalized contact lenses from Example A-2 are dried to the constant mass under reduced pressure. The lenses are then directly immersed into 1% by weight acetonitrile solution of the reactive photoinitiator prepared by the addition reaction from isophorone diisocyanate and 2-dimethylamino-2-benzyl-1-[4-(2-hydroxyethoxy)phenyl]-butan-1-one (synthesis see WO 96/20796(20 ml solution/lens). 3 drops of triethylamine (TEA) are then added to the solution. The amino groups on the lens surface react with the isocyanato groups of the photoinitiator molecules for 12 hours. After this time, the lenses are withdrawn from the reaction solution, 3x washed and extracted in acetonitrile for 6 hours and dried under reduced pressure for 2 hours. The dried lenses are subsequently used for photografting.

### Example C-1 (Acrylamide telomer (Mₙ 2000) synthesis

A 1000 mL round bottom flask is charged with a solution of 71.1 g (1 mol) Acrylamide, 4.93g (18.2 mmol) α,α'-azodiisobutyramidine dihydrochloride and 4.93 g (36.4 mmol) cysteaminhydrochloride in 400 ml of water. The clear and slightly yellowish solution is acidified with a few drops of hydrochloric acid to pH3. The stirred acidic solution is evacuated to 5000 Pa (50 mbar) and filled with argon. This is repeated three times. With a constant stream of Argon, this solution is poured into a 500 ml dropping funnel which is put onto an 'flow-through-reactor' consisting of an 1000ml three-necked round-bottom flask, reflux condenser, thermometer, magnetic stirrer and a 30 cm Liebig-condenser, which is filled with glass wool. The whole apparatus is constantly purged with argon. The dropping funnel is put onto the Liebig condenser, which is heated to 65°C. The flask is heated to 60°C. The solution is slowly dropped through the Liebig-condenser into the stirred flask. This takes 2.5 hrs. During this time the temperature in the flask is kept between 58-65°C. After the completed addition, the solution is stirred for 2hrs at 60°C.
NaOH is added to the clear and slightly yellowish solution until pH 10 is reached. The product is purified through reverse osmosis, using Millipore cartridge with a cut-off at 1000 Da and freeze-dried. A bright-white solid product is obtained (NH₂ 0.34mEq/g ,sulfur-value of the elemental analysis (0.33mEq/g); Mₙ 2000g/Mol).

### Example C-2 (Acrylamide telomer (Mₙ 1350) synthesis

A 1000 mL round bottom flask is charged with a solution of 99.5 g (1.46 mol) acrylamide, 1.27 g (4.68 mmol) α,α'-azodiisobutyramidine dihydrochloride and 15.9 g (0.14 mol) cysteaminhydrochloride in 300 ml of water. The clear and slightly yellowish solution is acidified with a few drops of hydrochloric acid (32%) to pH 3. The stirred acidic solution is evacuated to 5000 Pa (50 mbar) and filled with argon. This is repeated three times. With a constant stream of argon, this solution is poured into a 500 ml dropping funnel which is put onto an 'flow-through-reactor' consisting of an 1000ml three-necked round-bottom flask, reflux condenser, thermometer, magnetic stirrer and a 30 cm Liebig-condenser, which is filled with glass wool. The whole apparatus is constantly purged with argon. The dropping funnel is put onto the Liebig condenser, which is heated to 65°C. The flask is heated to 60°C. The solution is slowly dropped through the Liebig-condenser into the stirred flask. This takes 2 hrs. During this time the temperature in the flask is kept between 58-65°C. After the completed addition, the solution is stirred for 2 hrs at 60°C.
NaOH is added to the clear and slightly yellowish solution until pH 10 is reached. The product is purified through reverse osmosis, using Millipore cartridge with a cut-off at 1000 Da and then freeze-dried for 18 hrs.. A bright-white solid product is obtained (NH₂ 0.70mEq/g, sulfur-value of the elemental analysis (0.73mEq/g; Mₙ 1350g/Mol).

### Example C-3 (N,N-dimethyl acrylamide telomer (Mₙ 1850) synthesis

A 2000 mL round bottom flask is charged with a solution of 198.2 g (2 mol) N,N-dimethyl acrylamide, 2.72 g (10 mmol) α,α'-azodiisobutyramidine dihydrochloride and 24.8 g (0.22 mol) cysteaminhydrochloride in 600 ml of water. The clear and slightly yellowish solution is acidified with a few drops of Hydrochloric Acid (32%) to pH3. The stirred acidic solution is evacuated to 5000 Pa (50 mbar) and filled with argon. This is repeated three times.With a constant stream of argon, this solution is poured into a 1000 ml dropping funnel which is put onto an 'flow-through-reactor' consisting of an 1000ml three-necked round-bottom flask, reflux condenser, thermometer, magnetic stirrer and a 30 cm Liebig-condenser, which is filled with glass wool. The whole apparatus is constantly purged with argon.
The dropping funnel is put onto the Liebig condenser, which is heated to 60°C. The flask is also heated to 60°C. The solution is slowly dropped through the Liebig-condenser into the stirred flask. This takes 2.5 hrs. During this time the temperature in the flask is kept between 58-65°C. After the completed addition, the solution is stirred for 2hrs at 60°C.30 % NaOH solution is added to the clear and slightly yellowish solution until pH 10 is reached. The product is purified through reverse osmosis, using Millipore cartridge with a cut-off at 1000 Da and freeze-dried. A bright-white solid product is obtained (NH₂ 0.54mEq/g; Mₙ ,~1850 g/Mol).

### Example D-1 (Preparation of IEM-functionalized acrylamide telomer solution)

7.5 g of acrylamide telomer with amino end group (amine titration = 0.70 mEq/g), prepared by Example C-2 are dissolved in 80 ml of HPLC water. Argon is then let to bubble through the solution for the period of about 30 minutes. This mixture is then added to the equimolar amount (0.81 g) of isocyanatoethyl methacrylate (IEM, isocyanate titration = 6.45 mEq/g) under stirring. The whole mixture is then stirred under argon flow for 12 hours. After adding of 0.8 g of NaCl to the solution and 10 minutes stirring, the mixture is filtered through 0.45 µ m Teflon filter, degassed by repeated (3x) evacuation and bubbling with argon in order to remove oxygen and used for photografting.

### Example D-2 (Preparation of IEM-functionalized N,N-dimethylacrylamide telomer solution)

5 g of N,N-dimethylacrylamide telomer with amino end group (amine titration = 0.53 mEq/g), prepared by Example C-3 are dissolved in 100 ml of HPLC water. Argon is then let to bubble through the solution for the period of about 30 minutes. This mixture is then added to the equimolar amount (0.41 g) of isocyanatoethyl methacrylate (IEM, isocyanate titration = 6.45 mEq/g) under stirring. The whole mixture is then stirred under argon flow for 12 hours. After adding of 1.0 g of NaCl to the solution and 10 minutes stirring, the mixture is filtered through 0.45 µm Teflon filter, degassed with nitrogen in order to remove oxygen and used for photografting.

### Example E-1 (Photografting of IEM-functionalized acrylamide telomers onto a contact lens surface

1 ml of the IEM-functionalized acrylamide telomer solution from Example D-1 is introduced into a small Petri dish of a volume of about 2 ml in a glove box. The dried lens from Example B-1, carrying covalently linked photoinitiator molecules on its surface, is then placed into this solution and an additional 0.5 ml of the degassed solution is added on the lens in order to cover the whole lens with the solution. After 10 minutes, the Petri dish with the lens in the solution is exposed to 14.5 mW/cm² ultraviolet light for a period of about 1.5 minutes.

The modified lens is then withdrawn from the solution, washed twice in destilled water, continuously extracted in ultra pure water for 16 h and analyzed by AFM, ATR-FTIR and contact angle measurements.
The thickness of the coating is in the range of 250-300 nm as determined by AFM.
Water/air contact angles on the modified lens are 0° adv., 0° rec., 0° hysteresis. In comparison, the contact angles of non-modified lens are 101° adv., 64° rec., 37° hysteresis. The lens held continuous water layer on the surface for over 1 minute.

### Example E-2 (Photografting of IEM-functionalized acrylamide telomers onto a contact lens surface)

Two lenses from Example B-1 are coated in accordance with Example E-1, but instead of 1.5 minutes of exposition, 1.7 minutes exposition time is used for photografting.
Water/air contact angles on the modified lenses are 0° adv., 0° rec., 0° hysteresis.

### Example E-3 Photografting of IEM-functionalized N,N-dimethylacrylamide telomers onto a contact lens surface

1 ml of the IEM-functionalized N,N-dimethylacrylamide telomer solution from Example D-2 is introduced into a small Petri dish of a volume of about 2 ml in a glove box. The dried lens from Example B-1, carrying covalently linked photoinitiator molecules on its surface, is then placed into this solution and an additional 0.5 ml of the degassed solution is added on the lens in order to cover the whole lens with the solution. After 10 minutes, the Petri dish with the lens in the solution is exposed to 14.5 mW/cm² ultraviolet light for a period of about 1.5 minutes. The lens is then turned over and the exposition is repeated by applying 14.5 mW/cm² UV light for an additional 1.5 minutes.
The modified lens is then withdrawn from the solution, washed twice in destilled water, continuously extracted in ultra pure water for 16 h and analyzed by AFM, ATR-FTIR and contact angle measurements.
The thickness of the coating is in the range of 300-400 nm as determined by AFM.
Water/air contact angles on the modified lens are 0° adv., 0° rec., 0° hysteresis. In comparison, the contact angles of a non-modified lens are 101° adv., 64° rec., 37° hysteresis.

### Example E-4 (Photografting of IEM-functionalized acryrlamide telomers onto the contact lens surface under ambient conditions)

In a laminar flow hood, 1 ml of the IEM-functionalized acrylamide telomer solution from Example D-1 is introduced into a small Petri dish of a volume of about 2 ml. The dried lens from Example B-1, carrying covalently linked photoinitiator molecules on its surface, is then placed into this solution and an additional 0.5 ml of the degassed solution is added on the lens in order to cover the whole lens with the solution. After 10 minutes, the Petri dish with the lens in the solution is exposed to 2.05 mW/cm² ultraviolet light (MACAM-UV-Lamp) for a period of 2.5 minutes. The modified lens is then withdrawn from the solution, washed twice in destilled water, continuously extracted in ultra pure water for 16 h and analyzed by Atomic Force Microscopy (AFM), Fourier Transform Infrared-Attenuated Total Reflection Mode (ATR-FTIR) and contact angle measurements.
The thickness of the coating is in the range of 500-600 nm as determined by AFM.
Water/air contact angles on the modified lens are 0° adv., 0° rec., 0° hysteresis. In comparison, the contact angles of non-modified lens are 101° adv., 64° rec., 37° hysteresis. The lens held continuous water layer on the surface for over 1 minute.

### Example E-5 (Photografting of IEM-functionalized N,N-dimethylacrylamide telomers onto

### the contact lens surface under ambient conditions)

In a laminar flow hood, 1 ml of the IEM-functionalized N,N-dimethylacrylamide telomer solution from Example D-2 is introduced into a small Petri dish of a volume of about 2 ml. The dried lens from Example B-1, carrying covalently linked photoinitiator molecules on its surface, is then placed into this solution and an additional 0.5 ml of the degassed solution is added on the lens in order to cover the whole lens with the solution. After 10 minutes, the Petri dish with the lens in the solution is exposed to 2.36 mW/cm² ultraviolet light (MACAM-UV-Lamp) for a period of 2.5 minutes. The modified lens is then withdrawn from the solution, washed twice in destilled water, continuously extracted in ultra pure water for 16 h and analyzed by AFM, ATR-FTIR and contact angle measurements.
Water/air contact angles on the modified lens are 6° adv., 0° rec., 6° hysteresis. In comparison, the contact angles of non-modified lens are 101° adv., 64° rec., 37° hysteresis.

### Example E-6 Photografting of IEM-functionalized acrylamide telomers onto the contact lens surface

1 mf of the IEM-functionalized acrylamide telomer solution from Example D-1 is introduced into a small Petri dish of a volume of about 2.5 ml in a glove box. The dried lens from Example B-2, carrying covalently linked photoinitiator molecules on its surface, is then placed into this solution and an additional 1 ml of the degassed solution is added on the lens in order to cover the whole lens with the solution. After 10 minutes, the Petri dish with the lens in the solution is exposed to 14.5 mW/cm² ultraviolet light for a period of about 3 minutes.
The modified lens is then withdrawn from the solution, washed twice in destilled water, continuously extracted in ultra pure water for 16 h and analyzed by ATR-FTIR and contact angle measurements.
Water/air contact angles on the modified lens are 24° adv., 16° rec., 8° hysteresis. In comparison, the contact angles of non-modified lens are 101° adv., 64° rec., 37° hysteresis.

## Claims

1. A composite material comprising
(a) an inorganic or organic bulk material that is devoid of ionic groups and has adsorbed or heteropolarly bound on its surface a polyionic material that comprises covalently bound initiator moieties for radical polymerization; and
(b) a hydrophilic surface coating obtainable by applying one or more different ethylenically unsaturated hydrophilic monomers or macromonomers to the bulk material surface provided with the initiator radicals and polymerizing said monomers or macromonomers.

2. A composite material according to claim 1, which is a biomedical device, preferably an ophthalmic device such as a contact lens, intraocular lens or artificial cornea.

3. A composite material according to claim 1 or 2, wherein the bulk material comprises an organic polymer selected from a polysiloxane, perfluoroalkyl polyether, fluorinated polyacrylate and -methacrylate and an amphiphilic segmented copolymer comprising at least one polysiloxane or perfluoroalkyl polyether segment and at least one hydrophilic segment

4. A composite material according to any one of claims 1 to 3, wherein the polyionic material consists of one ionic polymer.

5. A composite material according to any one of claims 1 to 3, wherein the polyionic material includes at least one bilayer, the bilayer comprising a first ionic polymer and a second ionic polymer having charges opposite of the charges of the first ionic polymer.

6. A composite material according to claim 5, wherein the bilayer(s) comprise(s) an anionic polymer selected from a linear polyacrylic acid, a branched polyacrylic acid, a polymethacrylic acid, a polyacrylic acid or polymethacrylic acid copolymer, a maleic or fumaric acid copolymer, a poly(styrenesulfonic acid), a polyamido acid, a poly(2-acrylamido-2-methylpropanesulfonic acid), and an alkylene polyphosphate, alkylene polyphosphonate, carbohydrate polyphosphate or carbohydrate polyphosphonate; and a cationic polymer selected from a polyallylamine (PAH); a polyethyleneimine (PEI); a polyvinylamine homo- or copolymer; a poly(vinylbenzyl-tri-C₁-C₄-alkylammonium salt); a polymer of an aliphatic or araliphatic dihalide and an aliphatic N,N,N',N'-tetra-C₁-C₄-alkyl-alkylenediamine; a poly(vinylpyridin) or poly(vinylpyridinium salt); a poly (N,N-diallyl-N,N-di-C₁-C₄-alkylammoniumhalide); a homo- or copolymer of a quatemized di-C₁-C₄-alkyl-aminoethyl acrylate or methacrylate; POLYQUAD®; and a polyaminoamide.

7. A composite material according to claim 5 or 6, wherein the bilayer(s) comprise(s) an anionic polymer selected from a linear or branched polyacrylic acid and an acrylic acid copolymer; and a cationic polymer selected from a polyallylamine homopolymer; a polyallylamine comprising modifier units of the formula 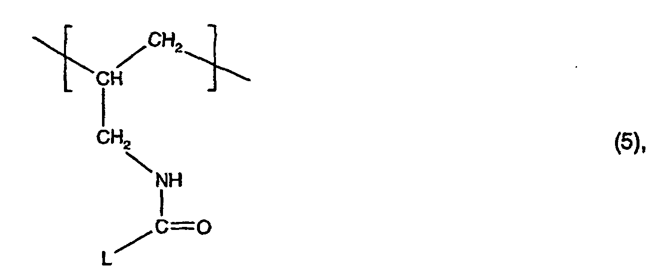 wherein L is C₂-C₆-alkyl which is substituted by two or more same or different substituents selected from the group consisting of hydroxy, C₂-C₅-alkanoyloxy and C₂-C₅-alkylaminocarbonyloxy; a polyvinylamine homo- or -copolymer; and a polyethyleneimine homopolymer.

8. A composite material according to any one of claims 5 to 7, wherein the bilayer(s) comprise(s) a first anionic polymer and a second cationic polymer.

9. A composite material according to any one of claims 5 to 8, wherein the bilayer(s) are formed on the bulk material surface by a dip method involving the steps of
(i) applying a coating of a first ionic polymer to the bulk material by immersing the bulk material in a solution of the first ionic polymer; and
(ii) applying a coating of a second ionic polymer having charges opposite of the charges of the first ionic polymer to the bulk material by immersing the bulk material in a solution of the second ionic polymer.

10. A composite material according to any one of claims 5 to 9, wherein the bulk material comprises one or more bilayers having -NH₂ and/or -NH- groups attached to its surface, some of whose H atoms have been substituted by radicals of the formulae 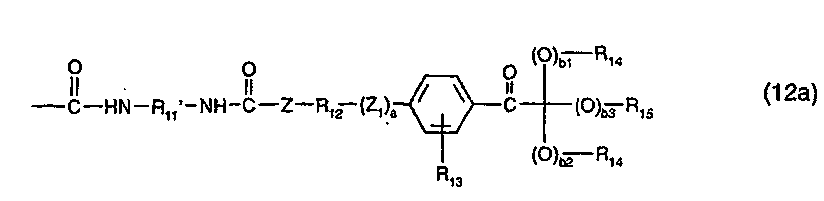 or 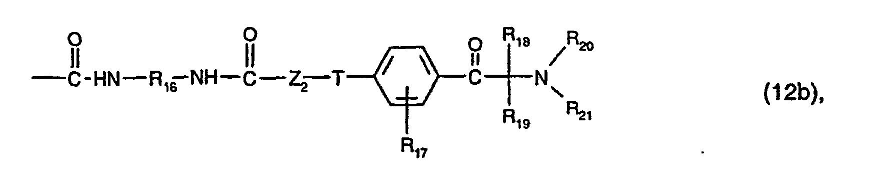 wherein Z is bivalent -O-, -NH- or -NR₂₂-; Z₁ is -O-, -O-(O)C-, -C(O)-O- or -O-C(O)-O-; R₁₃ is H, C₁-C₁₂-alkyl, C₁-C₁₂-alkoxy or N-C₁-C₁₂-alkylamino; R₁₄ and R₁₅ are each independently of the other H, linear or branched C₁-C₈-alkyl, C₁-C₈-hydroxyalkyl or C₆-C₁₀-aryl, or the groups R₁₄-(O)_{b1}- and R₁₄-(O)_{b2}- together are -(CH₂)_{c}- wherein c is an integer from 3 to 5, or the groups R₁₄-(O)_{b1}-, R₁₄-(O)_{b2}- and R₁₅-(O)_{b3}- together are a radical of the formula 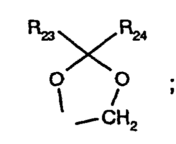 R₁₂ is a direct bond or linear or branched C₁-C₈-alkylene that is unsubstituted or substituted by -OH and/or is uninterrupted or interrupted by one or more groups -O-, -O-C(O)- or -O-C(O)-O-; R₁₁' is branched C₃-C₁₈-alkylene, unsubstituted or C₁-C₄-alkyl- or C₁-C₄-alkoxy-substituted C₆-C₁₀-arylene, or unsubstituted or C₁-C₄-alkyl- or C₁-C₄-alkoxy-substituted C₇-C₁₈-aralkylene, unsubstituted or C₁-C₄-alkyl- or C₁-C₄-alkoxy-substituted C₃-C₈-cycloalkylene, unsubstituted or C₁-C₄-alkyl- or C₁-C₄-alkoxy-substituted C₃-C₈-cycloalkylene-C_{y}H_{2y}- or unsubstituted or C₁-C₄-alkyl- or C₁-C₄-alkoxy-substituted -C_{y}H_{2y}-(C₃-C₈-cycloalkylene)-C_{y}H_{2y}- wherein y is an integer from 1 to 6; R₁₆ independently has the same definitions as R₁₁' or is linear C₃-C₁₈-alkylene; R₂₂ is linear or branched C₁-C₆-alkyl; T is bivalent -O-, -NH-, -S-, C₁-C₈-alkylene or 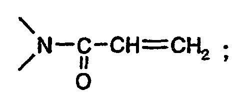 Z₂ is a direct bond or -O-(CH₂)_{d}- wherein d is an integer from 1 to 6 and the terminal CH₂ group of which is linked to the adjacent T in formula (12b); R₁₇ is H, C₁-C₁₂-alkyl, C₁-C₁₂-alkoxy, N-C₁-C₁₂-alkylamino or -NR₂₅R₂₆ wherein R₂₅ is C₁-C₈-alkyl and R₂₆ is H or C₁-C₈-alkyl; R₁₈ is linear or branched C₁-C₈-alkyl, C₂-C₈-alkenyl or C₆-C₁₀-aryl-C₁-C₈-alkyl; R₁₉ independently of R₁₈ has the same definitions as R₁₈ or is C₆-C₁₀-aryl, or R₁₈ and R₁₉ together are -(CH₂)ₑ- wherein e is an integer from 2 to 6; R₂₀ and R₂₁ are each independently of the other linear or branched C₁-C₈-alkyl that may be substituted by C₁-C₄-alkoxy, or C₆-C₁₀-aryl-C₁-C₈-alkyl or C₂-C₈-alkenyl; or R₂₀ and R₂₁ together are -(CH₂)_{f1}-Z₃-(CH₂)_{f2}- wherein Z₃ is a direct bond, -O-, -S- or -NR₂₆-, and R₂₆ is H or C₁-C₈-alkyl and f1 and f2 are each independently of the other an integer from 2 to 4; R₂₃ and R₂₄ are each independently of the other H, C₁-C₈-alkyl, C₃-C₈-cycloalkyl, benzyl or phenyl; and a, b1, b2 and b3 are each independently of the other 0 or 1; subject to the provisos that b1 and b2 are each 0 when R₁₅ is H; that the total of (b1+b2+b3) is not exceeding 2; and that a is 0 when R₁₂ is a direct bond.

11. A composite material according to any one of claims 1 to 10, wherein according to (b) a hydrophilic macromonomer of the formula 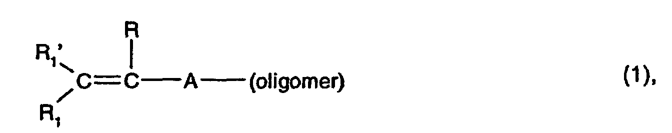 is applied, wherein R₁ is hydrogen, C₁-C₆-alkyl or a radical -COOR';
R, R' and R₁' are each independently of the other hydrogen or C₁-C₆-alkyl;
A is a direct bond or is a radical of formula
-C(O)-(A₁)ₙ-X- (2a)
or
-(A₂)ₘ-NH-C(O)-X- (2b);
or
-(A₂)ₘ-X-C(O)- (2c);
or
-C(O)-NH-C(O)-X- (2d);
or
-C(O)-X₁-(alk*)-X-C(O)- (2e);
or
A and R₁, together with the adjacent double bond, are a radical of formula 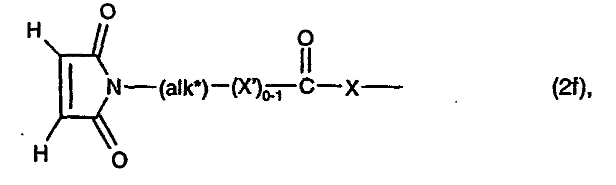 A₁ is -O-C₂-C₁₂-alkylene which is unsubstituted or substituted by hydroxy, or is -O-C₂-C₁₂-alkylene-NH-C(O)- or -O-C₂-C₁₂-alkylene-O-C(O)-NH-R₁₁-NH-C(O)-, wherein R₁₁ is linear or branched C₁-C₁₈-alkylene or unsubstituted or C₁-C₄-alkyl- or C₁-C₄-alkoxy-substituted C₆-C₁₀-arylene, C₇-C₁₈-aralkylene, C₆-C₁₀-arylene-C₁-C₂-alkylene-C₆-C₁₀-arylene, C₃-C₈-cycloalkylene, C₃-C₈-cycloalkylene-C₁-C₆-alkylene, C₃-C₈-cycloalkylene-C₁-C₂-alkylene-C₃-C₈-cycloalkylene or C₁-C₆-alkylene-C₃-C₈-cycloalkylene-C₁-C₆-alkylene ;
A₂ is C₁-C₈-alkylene; phenylene or benzylene;
m and n are each independently of the other the number 0 or 1;
X, X₁ and X' are each independently of the other a bivalent group -O- or -NR", wherein R" is hydrogen or C₁-C₆-alkyl;
(alk*) is C₂-C₁₂-alkylene;
and (oligomer) denotes
(i) the radical of a telomer of formula 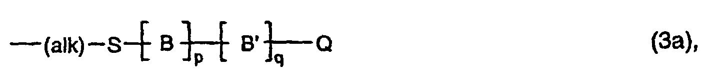 wherein (alk) is C₂-C₁₂-alkylene,
Q is a monovalent group that is suitable to act as a polymerization chain-reaction terminator,
p and q are each independently of another an integer from 0 to 250, wherein the total of (p+q) is an integer from 2 to 250,
and B and B' are each independently of the other a 1,2-ethylene radical derivable from a copolymerizable vinyl monomer by replacing the vinylic double bond by a single bond, at least one of the radicals B and B' being substituted by a hydrophilic substituent; or
(ii) the radical of an oligomer of the formula 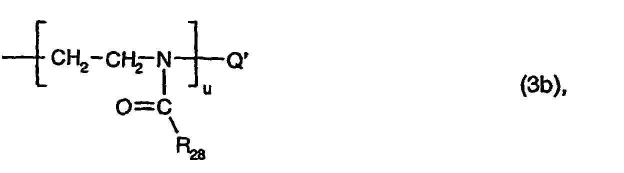 wherein R₂₈ is hydrogen or unsubstituted or hydroxy-substituted C₁-C₁₂-alkyl, u is an integer from 2 to 250 and Q' is a radical of a polymerization initiator; or
(iii) the radical of formula 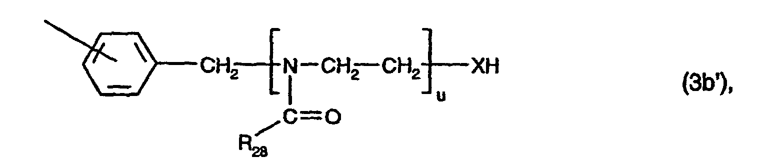 wherein R₂₈, X and u are as defined above, or
(iv) the radical of an oligomer of formula 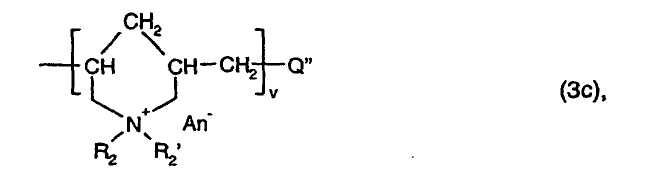 wherein R₂ and R₂' are each independently C₁-C₄-alkyl, An⁻ is an anion, v is an integer from 2 to 250, and Q" is a monovalent group that is suitable to act as a polymerization chain-reaction terminator; or
(v) the radical of an oligopeptide of formula
-(CHR₄-C(O)-NH)ₜ-CHR₄-COOH (3d)
or
-CHR₄-(NH-C(O)-CHR₄)ₜ-NH₂ (3d'),
wherein R₄ is hydrogen or C₁-C₄-alkyl which is unsubstituted or substituted by hydroxy, carboxy, carbamoyl, amino, phenyl, o-, m- or p-hydroxyphenyl, imidazolyl, indolyl or a radical -NH-C(=NH)-NH₂ and t is an integer from 2 to 250, or the radical of an oligopeptide based on proline or hydroxyproline; or
(vi) the radical of a polyalkylene oxide of formula
-(alk**-O)_{z}-[CH₂-CH₂-O]ᵣ-[CH₂-CH(CH₃)-O]ₛ-R₃₀ (3e),
wherein R₃₀ is hydrogen or C₁-C₂₄-alkyl, (alk**) is C₂-C₄-alkylene, z is 0 or 1, r and s are each independently an integer from 0 to 250 and the total of (r+s) is from 2 to 250; or
(vii) the radical of an oligosaccharide;
subject to the provisos that
A is not a direct bond if (oligomer) is a radical of formula (3a);
A is a direct bond if (oligomer) is a radical of formula (3b');
A is not a radical of formula (2c) or (2e) if (oligomer) is a radical of formula (3b), (3c), (3d), (3e) or is the radical of an oligosaccharide;and
A is a radical of formula (2c) or (2e) if (oligomer) is a radical of formula (3d').

12. A composite material according to claim 11, wherein the hydrophilic macromonomer is a compound of formula (1), wherein R is hydrogen or methyl, R₁ is hydrogen, methyl or carboxyl, R₁' is hydrogen, A is a radical of the formula (2a) or (2b), and (oligomer) is the radical of a telomer of formula (3a).

13. A composite material according to claim 11 or 12, wherein (oligomer) denotes a radical of formula (3a), and the radical -(alk)-S-[B]ₚ-[B']_{q}-Q is a radical of formula 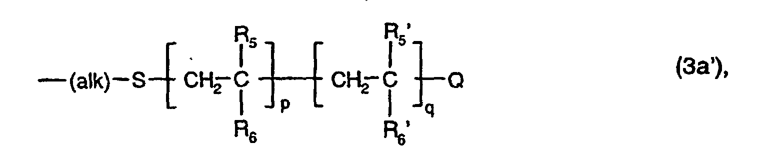 wherein (alk) is C₂-C₄-alkylene, R₅ and R₅' are each independently hydrogen or methyl, Q is a monovalent group that is suitable to act as a polymerization chain-reaction terminator, p and q are each independently an integer from 0 to 100 wherein the total of (p+q) is an integer from 5 to 100, and R₆ and R₆' are each independently a radical -COOY, wherein Y is C₁-C₂-alkyl, C₂-C₃-alkyl, which is substituted by hydroxy, amino or N,N-di-C₁-C₂-alkylamino, or is a radical -C₂-C₄-alkylene-NH-C(O)-O-G wherein -O-G is the radical of trehalose; a radical -CO-NY₁Y₂, wherein Y₁ and Y₂ are each independently of the other hydrogen or C₁-C₂-alkyl which is unsubstituted or substituted by hydroxy, or Y₁ and Y₂ together with the adjacent N-atom form a N-C₁-C₂-alkylpiperazino or morpholino ring; a heterocyclic radical selected from the group consisting of N-pyrrolidonyl, 2- or 4-pyridinyl, 2-methylpyridin-5-yl, 2-, 3- oder 4-hydroxypyridinyl, N-ε-caprolactamyl, N-imidazolyl, 2-methylimidazol-1-yl, N-morpholinyl and 4-N-methylpiperazin-1-yl; -COOH; -SO₃H; o-, m- or p-sulfophenyl; o-, m- or p-sulfomethylphenyl; a radical -CONY₅Y₆ wherein Y₅ is C₂-C₄-alkyl substituted by sulfo, and Y₆ is hydrogen; C₁-C₄-alkyl which is substituted by -NR₉R₉'R₉"⁺An⁻ wherein R₉, R₉' and R₉" are each independently of another hydrogen or C₁-C₄-alkyl and An⁻ is an anion; a radical -C(O)OY₇ wherein Y₇ is C₂-C₄-alkyl, which is substituted by -NR₉R₉'R₉"⁺An⁻ and is further unsubstituted or substituted by hydroxy, wherein R₉, R₉', R₉" and ⁺An⁻ are as defined; and a radical -C(O)O-CH₂-CH(OY₈)-CH₂-O-PO₂⁻-(CH₂)₂-N(CH₃)₃⁺, wherein Y₈ is hydrogen or the acyl radical of a higher fatty acid.

14. A composite material according to any one of claims 11 to 13, wherein the hydrophilic macromonomer applied according to (b) is of the formula 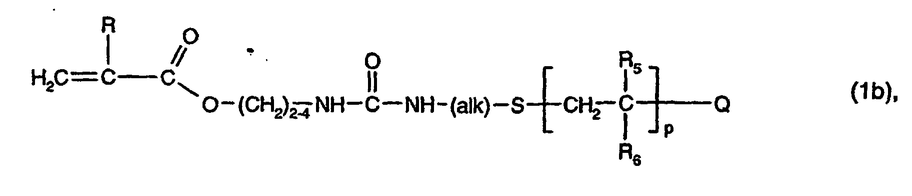 wherein R is hydrogen or methyl, (alk) is C₂-C₄-alkylene, R₅ is hydrogen or methyl, p is an integer of 5 to 50, Q is as defined, and R₆ is a radical -CONH₂, -CON(CH₃)₂, -CONH-(CH₂)₂-OH, 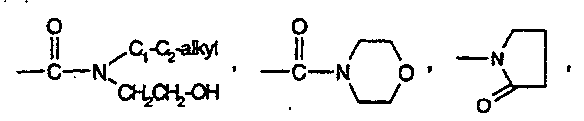 -COO-(CH₂)₂-N(CH₃)₂, or -COO(CH₂)₂₋₄-NHC(O)-O-G wherein -O-G is the radical of trehalose.

15. A composite material according to any one of claims 1 to 14, wherein the polymerization of the monomers or macromonomers on the modified bulk material surface is initiated by the action of irradiation.

16. A composite material according to claim 15, wherein the polymerization is initiated by the action of UV radiation for a time period of 0.5 to 5 minutes.

17. A composite material according to any of claims 1 to 16, wherein the hydrophilic surface coating (b) is obtainable by grafting at least one macromonomer to the bulk material surface with formation of a bottle-brush-type structure composed of tethered chains.

18. A composite material according to any of claims 1 to 17, wherein the hydrophilic surface coating has a coating thickness of from 0.01 to 5 0 µm, and preferably from 0.1 to 1 µm.

19. A process for the manufacture of a composite material, which comprises the steps:
(a) providing an inorganic or organic bulk material that is devoid of ionic groups and has adsorbed or heteropolarly bound on its surface a polyionic material that comprises covalently bound initiator moieties for radical polymerization;
(b) applying a coating of one or more different ethylenically unsaturated hydrophilic monomers or macromonomers to the bulk material surface provided with the initiator radicals, and
(c) polymerizing the coating of unsaturated hydrophilic macromonomers thermally or by irradiation, preferably by UV radiation.

20. A process according to claim 19, wherein the polyionic material includes at least one bilayer, the bilayer comprising a first ionic polymer and a second ionic polymer having charges opposite of the charges of the first ionic polymer, said bilayer being applied to the bulk material surface by a dip method comprising the steps of (i) immersing the bulk material in a solution of the first ionic polymer; and then (ii) immersing the bulk material in a solution of the second ionic polymer having charges opposite to the charges of the first ionic polymer.

21. A process according to claim 20, wherein the first ionic polymer is an anionic polymer comprising carboxy groups or a salt thereof, and the second ionic polymer is a cationic polymer comprising primary or secondary amino groups or a salt thereof.

22. A process according to any one of claims 19 to 21, wherein the initiator moieties for radical polymerization are bound to the polyionic material by reaction of amino groups of the polyionic material with isocyanato groups of the initiator moiety.

23. A process according to any one of claims 19 to 22, wherein the inorganic or organic bulk material is a biomedical device, particularly a contact lens, intraocular lens or artificial cornea.

24. Biomedical device comprising a composite material according to any one of claims 1 to 18.

25. Biomedical device according to claim 24, wherein the biomedical device is a contact lens, intraocular lens or artificial cornea.

26. Use of a composite material according to any of claims 1 to 18 for the manufacture of an ophthalmic device, particularly for the manufacture of a contact lens, intraocular lens or artificial cornea.

## Patentansprüche

1. Verbundwerkstoffmaterial, umfassend
(a) ein anorganisches oder organisches Massematerial, das frei von ionischen Gruppen ist und an seiner Oberfläche ein polyionisches Material, das kovalent gebundene Startereinheiten zur radikalischen Polymerisation umfasst, adsorbiert oder heteropolar gebunden aufweist; und
(b) eine hydrophile Oberflächenbeschichtung, die durch Auftragen von einem oder mehreren verschiedenen ethylenisch ungesättigten hydrophilen Monomeren oder Makromonomeren auf die Massematerialoberfläche, die mit den Starterradikalen versehen ist, und Polymerisieren der Monomere und Makromonomere erhältlich ist.

2. Verbundwerkstoffmaterial nach Anspruch 1, das eine biomedizinische Vorrichtung, vorzugsweise eine ophthalmische Vorrichtung, wie eine Kontaktlinse, Introkularlinse oder künstliche Cornea, darstellt.

3. Verbundwerkstoffmaterial nach Anspruch 1 oder 2, wobei das Massematerial ein organisches Polymer, ausgewählt aus einem Polysiloxan, Perfluoralkylpolyether, fluoriertem Polyacrylat und -methacrylat und einem amphiphilen segmentierten Copolymer, umfassend mindestens ein Polysiloxan- oder Perfluoralkylpolyethersegment und mindestens ein hydrophiles Segment, umfasst.

4. Verbundwerkstoffmaterial nach einem beliebigen der Ansprüche 1 bis 3, wobei das polyionische Material aus einem ionischen Polymer besteht.

5. Verbundwerkstoffmaterial nach einem beliebigen der Ansprüche 1 bis 3, wobei das polyionische Material mindestens eine Doppelschicht einschließt, wobei die Doppelschicht ein erstes ionisches Polymer und ein zweites ionisches Polymer mit Ladungen, entgegengesetzt zu den Ladungen des ersten ionischen Polymers, umfasst.

6. Verbundwerkstoffmaterial nach Anspruch 5, wobei die Doppelschicht(en) umfasst (umfassen) ein anionisches Polymer, ausgewählt aus einer linearen Polyacrylsäure, einer verzweigten Polyacrylsäure, einer Polymethacrylsäure, einem Polyacrylsäure- oder Polymethacrylsäure-Copolymer, einem Maleinsäure- oder Fumarsäure-Copolymer, einer Poly(styrolsulfonsäure), einer Polyamidosäure, einer Poly(2-acrylamido-2-methylpropansulfonsäure), und einem Alkylenpolyphosphat, Alkylenpolyphosphonat, Kohlenhydratpolyphosphat oder Kohlenhydratpolyphosphonat und ein kationisches Polymer, ausgewählt aus einem Polyallylamin (PAH); einem Polyethylenimin (PEI); einem Polyvinylamin-Homo- oder Copolymer; einem Poly(vinylbenzyl-tri-C₁-C₄alkylammoniumsalz); einem Polymer von einem aliphatischen oder araliphatischen Dihalogenid und einem aliphatischen N,N,N',N'-Tetra-C₁-C₄-alkyl-alkylendiamin; einem Poly(vinylpyridin) oder Poly(vinylpyridiniumsalz); einem Poly(N,N-diallyl-N,N-di-C₁-C₄-alkyl-ammoniumhalogenid); einem Homo- oder Copolymer eines quaternisierten Di-C₁-C₄-alkyl-aminoethylacrylat oder -methacrylat; POLYQUAD®; und einem Polyaminoamid.

7. Verbundwerkstoffmaterial nach Anspruch 5 oder 6, wobei die Doppelschicht(en) umfasst (umfassen) ein anionisches Polymer, ausgewählt aus einer linearen oder verzweigten Polyacrylsäure und einem Acrylsäure-Copolymer; und einem kationischen Polymer, ausgewählt aus einem Polyallylamin-Homopolymer; einem Polyallylamin, umfassend Modifizierungsmitteleinheiten der Formel 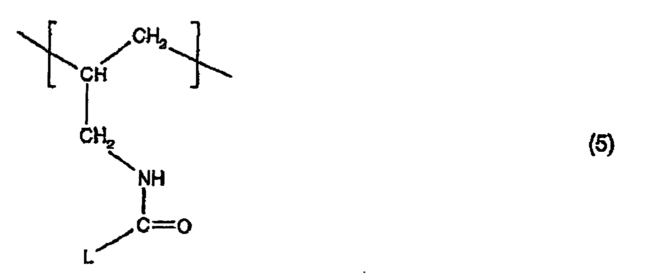 worin L C₂-C₆-Alkyl darstellt, das mit zwei oder mehreren gleichen oder verschiedenen Substituenten, ausgewählt aus der Gruppe, bestehend aus Hydroxy, C₂-C₅-Alkanoyloxy und C₂-C₅-Alkylamino-carbonyloxy substituiert ist; einem Polyvinylamin-Homo- oder -Copolymer; und einem Polyethylenimin-Homopolymer.

8. Verbundwerkstoffmaterial nach einem beliebigen der Ansprüche 5 bis 7, wobei die Doppelschicht(en) ein erstes anionisches Polymer und ein zweites kationisches Polymer umfasst (umfassen).

9. Verbundwerkstoffmaterial nach einem beliebigen der Ansprüche 5 bis 8, wobei die Doppelschicht(en) auf der Massematerialoberfläche durch ein Tauchverfahren gebildet werden, das die Schritte beinhaltet
(i) Auftragen einer Beschichtung auf ein erstes ionisches Polymer auf das Massematerial durch Eintauchen des Massematerials in eine Lösung des ersten ionischen Polymers; und
(ii) Auftragen einer Beschichtung eines zweiten ionischen Polymers mit Ladungen, entgegengesetzt zu den Ladungen des ersten ionischen Polymers auf das Massematerial durch Eintauchen des Massematerials in eine Lösung des zweiten ionischen Polymers.

10. Verbundwerkstoffmaterial nach einem beliebigen der Ansprüche 5 bis 9, wobei das Massematerial eine oder mehrere Doppelschichten umfasst, die an ihrer Oberfläche Gruppen -NH₂ und/oder -NH- gebunden aufweisen, wobei einige von ihren H-Atomen durch Reste der Formeln 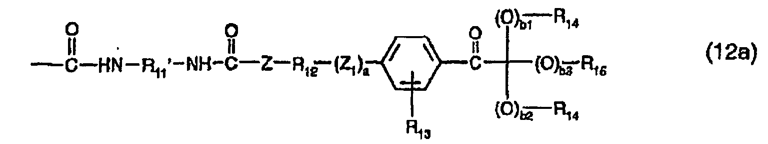 oder 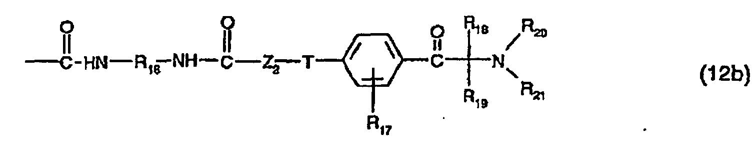 substituiert wurden, worin
Z zweiwertiges -O-, -NH- oder -NR₂₂- darstellt; Z₁ -O-, -O-(O)C-, -C(O)-O- oder -O-C(O)-O- darstellt; R₁₃ H, C₁-C₁₂-Alkyl, C₁-C₁₂-Alkoxy oder N-C₁-C₁₂-Alkylamino darstellt; R₁₄ und R₁₅ jeweils unabhängig voneinander H, lineares oder verzweigtes C₁-C₈-Alkyl, C₁-C₈-Hydroxyalkyl oder C₆-C₁₀-Aryl darstellen, oder die Gruppen R₁₄-(O)_{b1}und R₁₄-(O)_{b2}- zusammen -(CH₂)_{c}- sind, worin c eine ganze Zahl von 3 bis 5 ist oder die Gruppen R₁₄-(O)_{b1}-, R₁₄-(O)_{b2}- und R₁₅-(O)_{b3}- zusammen einen Rest der Formel 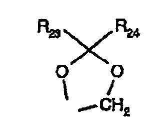 darstellen; R₁₂ eine direkte Bindung oder lineares oder verzweigtes C₁-C₈-Alkylen darstellt, das unsubstituiert oder mit -OH substituiert ist und/oder nicht unterbrochen oder durch eine oder mehrere Gruppen -O-, -O-C(O)- oder -O-C(O)-O- unterbrochen ist; R₁₁' verzweigtes C₃-C₁₈-Alkylen, unsubstituiertes oder C₁-C₄-Alkyl- oder C₁-C₄-Alkoxy-substituiertes C₆-C₁₀-Arylen oder unsubstituiertes oder C₁-C₄-Alkyl- oder C₁-C₄-Alkoxy-substituiertes C₇-C₁₈-Aralkylen, unsubstituiertes oder C₁-C₄-Alkyl- oder C₁-C₄-Alkoxy-substituiertes C₃-C₈-Cycloalkylen, unsubstituiertes oder C₁-C₄-Alkyl- oder C₁-C₄-Alkoxy-substituiertes C₃-C₈-Cycloalkylen-C_{y}H_{2y}- oder unsubstituiertes oder C₁-C₄-Alkyl- oder C₁-C₄-Alkoxysubstituiertes -C_{y}H_{2y}(C₃-C₈-Cycloalkylen)-C_{y}H_{2y}- darstellt, worin y eine ganze Zahl von 1 bis 6 ist; R₁₆ unabhängig die gleichen Definitionen wie R₁₁' aufweist oder lineares C₃-C₁₈-Alkylen darstellt; R₂₂ lineares oder verzweigtes C₁-C₆-Alkyl darstellt; T zweiwertiges -O-, -NH-, -S-, C₁-C₈-Alkylen oder 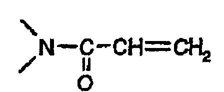 darstellt;
Z₂ eine direkte Bindung oder -O-(CH₂)_{d}- darstellt, worin d eine ganze Zahl von 1 bis 6 ist und die endständige Gruppe CH₂ davon an das benachbarte T in Formel (12b) gebunden ist; R₁₇ H, C₁-C₁₂-Alkyl, C₁-C₁₂-Alkoxy, N-C₁-C₁₂-Alkylamino oder -NR₂₅R₂₆ darstellt, worin R₂₅ C₁-C₈-Alkyl darstellt und R₂₆ H oder C₁-C₈-Alkyl darstellt; R₁₈ lineares oder verzweigtes C₁-C₈-Alkyl, C₂-C₈-Alkenyl oder C₆-C₁₀-Aryl-C₁-C₈-alkyl darstellt; R₁₉ unabhängig von R₁₈ die gleichen Definitionen wie R₁₈ aufweist oder C₆-C₁₀-Aryl darstellt, oder R₁₈ und R₁₉ zusammen -(CH₂)ₑ- darstellen, worin e eine ganze Zahl von 2 bis 6 ist; R₂₀ und R₂₁ jeweils unabhängig voneinander lineares oder verzweigtes C₁-C₈-Alkyl darstellen, das mit C₁-C₄-Alkoxy oder C₆-C₁₀-Aryl-C₁-C₈-alkyl oder C₂-C₈-Alkenyl substituiert sein kann; oder R₂₀ und R₂₁ zusammen -(CH₂)_{f1}-Z₃-(CH₂)_{f2}- darstellen, worin Z₃ eine direkte Bindung -O-, -S- oder -NR₂₆- darstellt und R₂₆ H oder C₁-C₈-Alkyl darstellt und f1 und f2 jeweils unabhängig voneinander eine ganze Zahl von 2 bis 4 sind; R₂₃ und R₂₄ jeweils unabhängig voneinander H, C₁-C₈-Alkyl, C₃-C₈-Cycloalkyl, Benzyl oder Phenyl darstellen; und a, b1, b2 und b3 jeweils unabhängig voneinander 0 oder 1 sind; unter den Maßgaben, dass b1 und b2 jeweils 0 sind, wenn R₁₅ H darstellt; dass die Gesamtheit von (bl+b2+b3) 2 nicht übersteigt und dass a 0 ist, wenn R₁₂ eine direkte Bindung darstellt.

11. Verbundwerkstoffmaterial nach einem beliebigen der Ansprüche 1 bis 10, wobei gemäß (b) ein hydrophiles Makromonomer der Formel 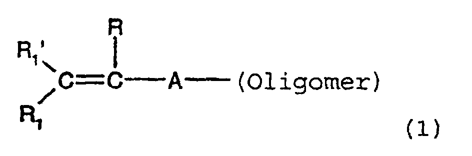 aufgetragen wird, worin R₁ Wasserstoff, C₁-C₆-Alkyl oder einen Rest -COOR' darstellt;
R, R' und R₁' jeweils unabhängig voneinander Wasserstoff oder C₁-C₆-Alkyl darstellen;
A eine direkte Bindung darstellt oder einen Rest der Formel
-C(O)-(A₁)ₙ-X- (2a)
oder
-(A₂)ₘ-NH-C(O)-X- (2b);
oder
-(A₂)ₘ-X-C(O)- (2c);
oder
-C(O)-NH-C(O)-X- (2d);
oder
-C(O)-X₁-(alk*)-X-C(O)- (2e)
darstellt; oder
A und R₁, zusammen mit der benachbarten Doppelbindung einen Rest der Formel 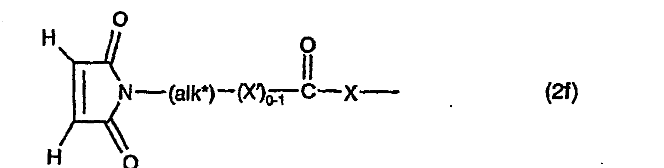 darstellen
A₁ -O-C₂-C₁₂-Alkylen darstellt, das unsubstituiert oder mit Hydroxy substituiert ist oder -O-C₂-C₁₂-Alkylen-NH-C(O)- oder -O-C₂-C₁₂-Alkylen-O-C(O)-NH-R₁₁-NH-C(O)- darstellt, worin
R₁₁ lineares oder verzweigtes C₁-C₁₈-Alkylen oder unsubstituiertes oder C₁-C₄-Alkyl- oder C₁-C₄-Alkoxy-substituiertes C₆-C₁₀-Arylen, C₇-C₁₈-Aralkylen, C₆-C₁₀-Arylen-C₁-C₂-alkylen-C₆-C₁₀-arylen, C₃-C₈-Cycloalkylen, C₃-C₈-Cycloalkylen-C₁-C₆-alkylen, C₃-C₈-Cycloalkylen-C₁-C₂-alkylen-C₃-C₈-cycloalkylen oder C₁-C₆-Alkylen-C₃-C₈cycloalkylen-C₁-C₆-alkylen darstellt;
A₂ C₁-C₈-Alkylen, Phenylen oder Benzylen darstellt;
m und n jeweils unabhängig voneinander die Zahl 0 oder 1 sind;
X, X₁ und X' jeweils unabhängig voneinander eine zweiwertige Gruppe -O- oder -NR" darstellen, worin R" Wasserstoff oder C₁-C₆-Alkyl darstellt;
(alk*) C₂-C₁₂-Alkylen darstellt;
und (Oligomer) bedeutet
(i) den Rest eines Telomers der Formel 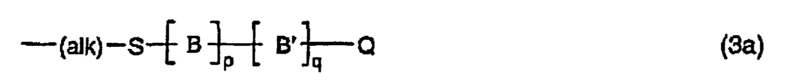 worin
(alk) C₂-C₁₂-Alkylen darstellt,
Q eine einwertige Gruppe darstellt, die geeignet ist, als ein Polymerisationskettenreaktionsstopper zu wirken,
p und q jeweils unabhängig voneinander eine ganze Zahl von 0 bis 250 sind, wobei die Gesamtheit von (p+q) eine ganze Zahl von 2 bis 250 ist,
und B und B' jeweils unabhängig voneinander einen 1,2-Ethylenrest, der von einem copolymerisierbaren Vinylmonomer durch Ersetzen der vinylischen Doppelbindung durch eine Einfachbindung ableitbar ist,
wobei mindestens einer der Reste B und B' mit einen hydrophilen Substituenten substituiert ist; oder
(ii) den Rest eines Oligomers der Formel 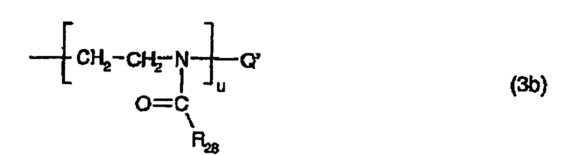 worin R₂₈ Wasserstoff oder unsubstituiertes oder Hydroxy-substituiertes C₁-C₁₂-Alkyl darstellt, u eine ganze Zahl von 2 bis 250 ist und Q' einen Rest eines Polymerisationsstarters darstellt; oder
(iii) den Rest der Formel 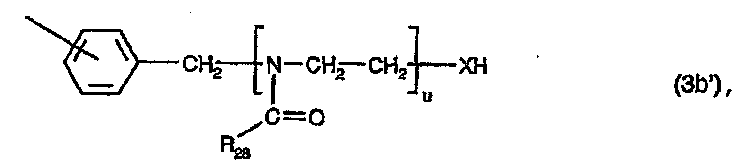 worin R₂₈, X und u wie vorstehend definiert sind oder
(iv) den Rest eines Oligomers der Formel 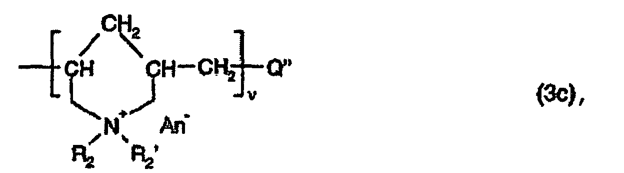 worin R₂ und R₂' jeweils unabhängig C₁-C₄-Alkyl darstellen, An⁻ ein Anion darstellt, v eine ganze Zahl von 2 bis 250 ist und Q" eine einwertige Gruppe darstellt, die geeignet ist, als Polymerisationskettenreaktionsstopper zu wirken; oder
(v) den Rest eines Oligopeptids der Formel
-(CHR₄-C(O)-NH)ₜ-CHR₄-COOH (3d)
oder
-CHR₄-(NH-C(O)-CHR₄)ₜ-NH₂ (3d'),
worin R₄ Wasserstoff oder C₁-C₄-Alkyl darstellt, das unsubstituiert oder mit Hydroxy, Carboxy, Carbamoyl, Amino, Phenyl, o-, m- oder p-Hydroxyphenyl, Imidazolyl, Indolyl oder einem Rest -NH-C(=NH)-NH₂ substituiert ist und t eine ganze Zahl von 2 bis 250 ist oder den Rest eines Oligopeptids, das auf Prolin oder Hydroxyprolin basiert; oder
(vi) den Rest eines Polyalkylenoxids der Formel
-(alk**-O)_{z}-[CH₂-CH₂-O]ᵣ-[CH₂-CH(CH₃)-O]ₛ-R₃₀ (3e),
worin R₃₀ Wasserstoff oder C₁-C₂₄-Alkyl darstellt, (alk**) C₂-C₄-Alkylen darstellt, z 0 oder 1 ist, r und s jeweils unabhängig eine ganze Zahl von 0 bis 250 sind und die Gesamtheit von (r+s) von 2 bis 250 ist; oder
(vii) den Rest eines Oligosaccharids; unter den Maßgaben, dass
A keine direkte Bindung darstellt, wenn (Oligomer) einen Rest der Formel (3a) darstellt;
A eine direkte Bindung darstellt, wenn (Oligomer) einen Rest der Formel (3b') darstellt;
A keinen Rest der Formel (2c) oder (2e) darstellt, wenn (Oligomer) einen Rest der Formel (3b), (3c), (3d), (3e) darstellt oder den Rest eines Oligosaccharids darstellt; und
A einen Rest der Formel (2c) oder (2e) darstellt, wenn (Oligomer) einen Rest der Formel (3d') darstellt.

12. Verbundwerkstoffmaterial nach Anspruch 11, wobei das hydrophile Makromonomer eine Verbindung der Formel (1) darstellt, worin R Wasserstoff oder Methyl darstellt, R₁ Wasserstoff, Methyl oder Carboxyl darstellt, R₁' Wasserstoff darstellt, A einen Rest der Formel (2a) oder (2b) darstellt, und (Oligomer) den Rest eines Telomers der Formel (3a) darstellt.

13. Verbundwerkstoffmaterial nach Anspruch 11 oder 12, wobei (Oligomer) einen Rest der Formel (3a) bedeutet und der Rest -(alk)-S-[B]ₚ-[B']_{q}-Q ein Rest der Formel 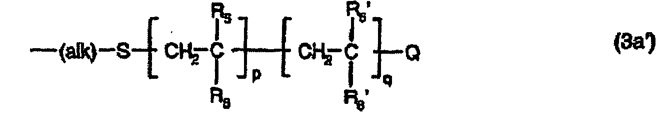 ist,
worin (alk) C₂-C₄-Alkylen darstellt, R₅ und R₅' jeweils unabhängig Wasserstoff oder Methyl darstellen, Q eine einwertige Gruppe darstellt, die geeignet ist, um als ein Polymerisationskettenreaktionsstopper zu wirken, p und q jeweils unabhängig eine ganze Zahl von 0 bis 100 sind, worin die Gesamtheit von (p+q) eine ganze Zahl von 5 bis 100 ist, und R₆ und R₆' jeweils unabhängig einen Rest -COOY, worin Y C₁-C₂-Alkyl, C₂-C₃-Alkyl, das mit Hydroxy, Amino oder N,N-Di-C₁-C₂-alkylamino substituiert ist, oder einen Rest -C₂-C₄-Alkylen-NH-C(O)-O-G darstellt, worin -O-G den Rest von Trehalose darstellt; einen Rest -CO-NY₁Y₂, worin Y₁ und Y₂ jeweils unabhängig voneinander Wasserstoff oder C₁-C₂-Alkyl darstellen, welches unsubstituiert oder mit Hydroxy substituiert ist, oder Y₁ und Y₂ zusammen mit dem benachbarten N-Atom einen N-C₁-C₂-Alkylpiperazin- oder Morpholinring bilden; einen heterocyclischen Rest, ausgewählt aus der Gruppe, bestehend aus N-Pyrrolidonyl, 2- oder 4-Pyridinyl, 2-Methylpyridin-5-yl, 2-, 3- oder 4-Hydroxypyridinyl, N-ε-Caprolactamyl, N-Imidazolyl, 2-Methylimidazol-1-yl, N-Morpholinyl und 4-N-Methylpiperazin-1-yl; -COOH; -SO₃H; o-, m- oder p-Sulfophenyl; o-, m- oder p-Sulfomethylphenyl; einen Rest -CONY₅Y₆, worin Y₅ C₂-C₄-Alkyl, substituiert mit Sulfo, darstellt und Y₆ Wasserstoff; C₁-C₄-Alkyl, das mit -NR₉R₉'R₉"⁺An⁻ substituiert ist, wobei R₉, R₉' und R₉" jeweils unabhängig voneinander Wasserstoff oder C₁-C₄-Alkyl darstellen und An⁻ ein Anion darstellt, darstellt; einen Rest -C(O)OY₇, worin Y₇ C₂-C₄-Alkyl darstellt, das mit -NR₉R₉'R₉"⁺An⁻ substituiert ist, und weiterhin unsubstituiert oder mit Hydroxy substituiert ist, wobei R₉, R₉', R₉" und ⁺An⁻ wie definiert sind und einen Rest -C (O)O-CH₂-CH(OY₈)-CH₂-O-PO₂⁻-(CH₂)₂-N(CH₃)₃⁺, worin Y₈ Wasserstoff oder den Acylrest einer höheren Fettsäure darstellt, darstellen.

14. Verbundwerkstoffmaterial nach einem beliebigen der Ansprüche 11 bis 13, wobei das hydrophile Makromonomer, das gemäß (b) aufgetragen wird, die Formel 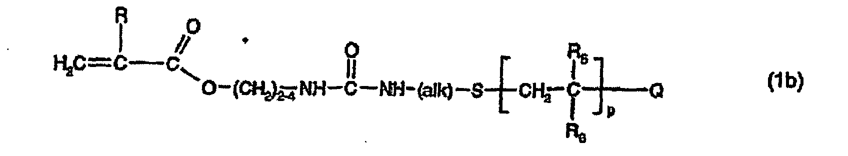 aufweist, worin R Wasserstoff oder Methyl darstellt, (alk) C₂-C₄-Alkylen darstellt, R₅ Wasserstoff oder Methyl darstellt, p eine ganze Zahl von 5 bis 50 ist, Q wie definiert ist und R₆ einen Rest -CONH₂, -CON(CH₃)₂, -CONH-(CH₂)₂-OH, 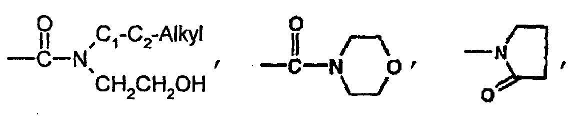 -COO-(CH₂)₂-N (CH₃)₂, oder -COO(CH₂)₂₋₄-NHC(O)-O-G darstellt, worin -O-G den Rest von Trehalose darstellt.

15. Verbundwerkstoffmaterial nach einem beliebigen der Ansprüche 1 bis 14, wobei die Polymerisation der Monomere oder Makromonomere auf der modifizierten Massematerialoberfläche durch die Wirkung von Bestrahlung gestartet wird.

16. Verbundwerkstoffmaterial nach Anspruch 15, wobei die Polymerisation durch die Wirkung von UV-Strahlung für einen Zeitraum von 0,5 bis 5 Minuten gestartet wird.

17. Verbundwerkstoffmaterial nach einem beliebigen der Ansprüche 1 bis 16, wobei die hydrophile Oberflächenbeschichtung (b) durch Pfropfen von mindestens einem Makromonomer auf die Massematerialoberfläche unter Bildung einer Struktur vom Flaschenbürstentyp, zusammengesetzt aus verknüpften Ketten, erhältlich ist.

18. Verbundwerkstoffmaterial nach einem beliebigen der Ansprüche 1 bis 17, wobei die hydrophile Oberflächenbeschichtung eine Beschichtungsdicke von 0,01 bis 50 µm und vorzugsweise 0,1 bis 1 µm aufweist.

19. Verfahren zur Herstellung eines Verbundwerkstoffmaterials, das die Schritte umfasst:
(a) Bereitstellen eines anorganischen oder organischen Massematerials, das von ionischen Gruppen frei ist und adsorbiert oder heteropolar gebunden an seine Oberfläche ein polyionisches Material aufweist, das kovalent gebundene Startereinheiten zur radikalischen Polymerisation umfasst;
(b) Auftragen einer Beschichtung von einem oder mehreren verschiedenen ethylenisch ungesättigten, hydrophilen Monomeren oder Makromonomeren auf die Massematerialoberfläche, die mit den Starterradikalen ausgestattet ist, und
(c) Polymerisieren der Beschichtung von ungesättigten hydrophilen Makromonomeren, thermisch oder durch Bestrahlung, vorzugsweise UV-Strahlung.

20. Verfahren nach Anspruch 19, wobei das polyionische Material mindestens eine Doppelschicht einschließt, wobei die Doppelschicht ein erstes ionisches Polymer und ein zweites ionisches Polymer mit Ladungen, entgegengesetzt zu den Ladungen des ersten ionischen Polymers, umfasst,
wobei die Doppelschicht auf die Massematerialoberfläche durch ein Tauchverfahren aufgetragen wird, umfassend die Schritte von
(i) Eintauchen des Massematerials in eine Lösung des ersten ionischen Polymers; und dann
(ii) Eintauchen des Massematerials in eine Lösung des zweiten ionischen Polymers mit Ladungen, entgegengesetzt zu den Ladungen des ersten ionischen Polymers.

21. Verfahren nach Anspruch 20, wobei das erste ionische Polymer ein anionisches Polymer, das Carboxygruppen umfasst oder ein Salz davon darstellt und das zweite ionische Polymer ein kationisches Polymer, das primäre oder sekundäre Aminogruppen umfasst oder Salz davon darstellt.

22. Verfahren nach einem beliebigen der Ansprüche 19 bis 21, wobei die Startereinheiten für die radikalische Polymerisation an das polyionische Material durch Reaktion der Aminogruppen des polyionischen Materials mit Isocyanatgruppen der Startereinheit gebunden sind.

23. Verfahren nach einem beliebigen der Ansprüche 19 bis 22, worin das anorganische oder organische Massematerial eine biomedizinische Vorrichtung, insbesondere eine Kontaktlinse, Intraokularlinse oder künstliche Cornea darstellt.

24. Biomedizinische Vorrichtung, umfassend ein Verbundwerkstoffmaterial nach einem beliebigen der Ansprüche 1 bis 18.

25. Biomedizinische Vorrichtung nach Anspruch 24, worin die biomedizinische Vorrichtung eine Kontaktlinse, Intraokularlinse oder künstliche Cornea darstellt.

26. Verwendung eines Verbundwerkstoffmaterials nach einem beliebigen der Ansprüche 1 bis 18 zur Herstellung einer ophthalmischen Vorrichtung, insbesondere zur Herstellung einer Kontaktlinse, Intraokularlinse oder künstlichen Cornea.

## Revendications

1. Matériau composite comprenant :
(a) un matériau en masse inorganique ou organique qui est dépourvu de groupes ioniques et présente à sa surface une matière polyionique adsorbée ou liée de manière hétéropolaire, qui comprend des groupements initiateurs liés de manière covalente pour une polymérisation radicalaire ; et
(b) un revêtement superficiel hydrophile susceptible d'être obtenu en appliquant un ou plusieurs monomère(s) ou macromonomère(s) hydrophile(s) à insaturation éthylénique à la surface du matériau en masse pourvu des radicaux initiateurs et en polymérisant lesdits monomères ou macromonomères.

2. Matériau composite selon la revendication 1, qui est un dispositif biomédical, de préférence un dispositif ophtalmique tel qu'une lentille de contact, une lentille intraoculaire ou une cornée artificielle.

3. Matériau composite selon la revendication 1 ou 2, dans lequel le matériau en masse comprend un polymère organique choisi entre un polysiloxane, un perfluoroalkylpolyéther, un polyacrylate et -méthacrylate fluoré, et un copolymère segmenté amphiphile comprenant au moins un segment polysiloxane ou perfluoroalkylpolyéther, et au moins un segment hydrophile.

4. Matériau composite selon l'une quelconque des revendications 1 à 3, dans lequel la matière polyionique est constituée d'un polymère ionique.

5. Matériau composite selon l'une quelconque des revendications 1 à 3, dans lequel la matière polyionique comprend au moins une bicouche, la bicouche comprenant un premier polymère ionique et un second polymère ionique ayant des charges opposées aux charges du premier polymère ionique.

6. Matériau composite selon la revendication 5, dans lequel la(les) bicouche(s) comprend(nent) un polymère anionique choisi entre un acide polyacrylique linéaire, un acide polyacrylique ramifié, un acide polyméthacrylique, un copolymère d'acide polyacrylique ou d'acide polyméthacrylique, un copolymère d'acide maléique ou fumarique, un acide poly(styrènesulfonique), un polyamidoacide, un acide poly(2-acrylamido-2-méthylpropanesulfonique) et un polyphosphate d'alkylène, un polyphosphonate d'alkylène, un polyphosphate d'hydrate de carbone ou un polyphosphonate d'hydrate de carbone ; et un polymère cationique choisi entre une polyallylamine (PAH) ; une polyéthylèneimine (PEI) ; un homo- ou copolymère de polyvinylamine ; un sel de poly(vinylbenzyl-tri-alkyle en C₁ à C₄-ammonium) ; un polymère d'un dihalogénure aliphatique ou araliphatique et une N,N,N',N'-tétra-alkyle en C₁ à C₄-alkylènediamine aliphatique ; un sel de poly(vinylpyridine) ou de poly(vinylpyridinium) ; un poly(halogénure de N,N-diallyl-N,N-di-alkyle en C₁ à C₄-ammonium) ; un homo- ou copolymère d'un acrylate ou méthacrylate de di-alkyle en C₁ à C₄-aminoéthyle quaternisé ; le POLYQUAD® ; et un polyaminoamide.

7. Matériau composite selon la revendication 5 ou 6, dans lequel la(les) bicouche(s) comprend(nent) un polymère anionique choisi entre un acide polyacrylique linéaire ou ramifié et un copolymère d'acide acrylique ; et un polymère cationique choisi entre un homopolymère de polyallylamine ; une polyallylamine comprenant des motifs modificateurs de formule : 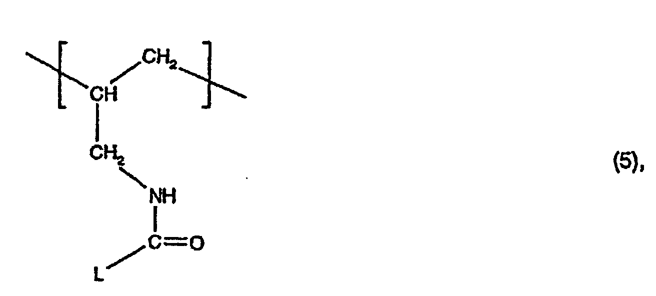 dans laquelle L est un groupe alkyle en C₂ à C₆ qui est substitué par deux ou plusieurs substituants identiques ou différents choisis dans le groupe constitué parmi les groupes hydroxy, alcanoyloxy en C₂ à C₅ et alkyle en C₂ à C₅-aminocarbonyloxy ; un homo- ou -copolymère de polyvinylamine ; et un homopolymère de polyéthylèneimine.

8. Matériau composite selon l'une quelconque des revendications 5 à 7, dans lequel la(les) bicouche(s) comprend(nent) un premier polymère anionique et un second polymère cationique.

9. Matériau composite selon l'une quelconque des revendications 5 à 8, dans lequel la(les) bicouche(s) est(sont) formée(s) à la surface du matériau en masse par un procédé d'immersion faisant intervenir les étapes consistant à :
(i) appliquer un revêtement d'un premier polymère ionique au matériau en masse en immergeant le matériau en masse dans un solution du premier polymère ionique ; et
(ii) appliquer un revêtement d'un second polymère ionique ayant des charges opposées aux charges du premier polymère ionique au matériau en masse en immergeant le matériau en masse dans une solution du second polymère ionique.

10. Matériau composite selon l'une quelconque des revendications 5 à 9, dans laquelle le matériau en masse comprend une ou plusieurs bicouches ayant des groupes -NH₂ et/ou -NH- fixés à sa surface, les atomes de H de certains d'entre eux ayant été substitués par des radicaux de formules : 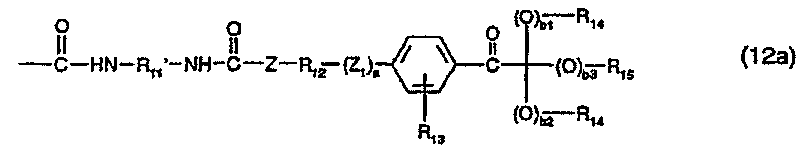 ou 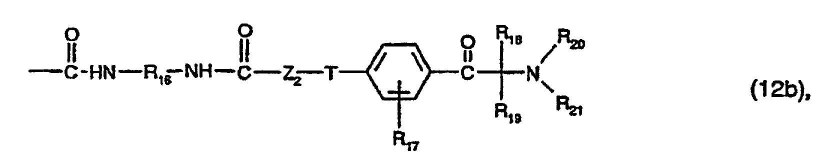 dans lesquelles Z est un groupe bivalent -O-, -NH- ou -NR₂₂- ; Z₁ est un groupe -O-, -O-(O)C-, -C(O)-O- ou -O-C(O)-O- ; R₁₃ représente H ou un groupe alkyle en C₁ à C₁₂, alcoxy en C₁ à C₁₂ ou N-alkyle en C₁ à C₁₂-amino ; R₁₄ et R₁₅ représentent chacun indépendamment l'un de l'autre H, des groupes alkyle en C₁ à C₈, linéaires ou ramifiés, hydroxyalkyle en C₁ à C₈ ou aryle en C₆ à C₁₀, ou les groupes R₁₄-(O)_{b1}- et R₁₄-(O)_{b2}- représentent ensemble un groupe -(CH₂)_{c}-, où c est un nombre entier de 3 à 5, ou les groupes R₁₄-(O)_{b1}-, R₁₄-(O)_{b2}- et R₁₅-(O)_{b3}- représentent ensemble un radical de formule : 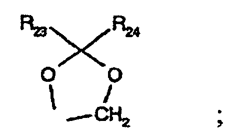 R₁₂ est une liaison directe ou un groupe alkylène en C₁ à C₈ linéaire ou ramifié, qui est non substitué ou substitué par un groupe -OH et/ou est ininterrompu ou interrompu par un ou plusieurs groupes -O-, -O-C(O)- ou -O-C(O)-O- ; R₁₁' est un groupe alkylène en C₃ à C₁₈ ramifié, un groupe arylène en C₆ à C₁₀ non substitué ou substitué par un groupe alkyle en C₁ à C₄ ou alcoxy en C₁ à C₄, ou un groupe aralkylène en C₇ à C₁₈ non substitué ou substitué par un groupe alkyle en C₁ à C₄ ou alcoxy en C₁ à C₄, cycloalkylène en C₃ à C₈ non substitué ou substitué par un groupe alkyle en C₁ à C₄ ou alcoxy en C₁ à C₄, cycloalkylène en C₃ à C₈-C_{y}H_{2y}- non substitué ou substitué par un groupe alkyle en C₁ à C₄ ou alcoxy en C₁ à C₄, ou C_{y}H_{2y}-(cycloalkylène en C₃ à C₈)-C_{y}H_{2y}- non substitué ou substitué par un groupe alkyle en C₁ à C₄ ou alcoxy en C₁ à C₄, où y est un nombre entier de 1 à 6 ; R₁₆ a indépendamment les mêmes significations que R₁₁' ou est un groupe alkylène linéaire en C₃ à C₁₈ ; R₂₂ est un groupe alkyle en C₁ à C₆ linéaire ou ramifié ; T est un groupe bivalent -O-, -NH-, -S-, alkylène en C₁ à C₈ ou 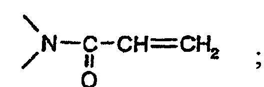 Z₂ est une liaison directe ou un groupe -O-(CH₂)_{d}- où d est un nombre entier de 1 à 6 et dont le groupe CH₂ terminal est lié au radical T adjacent dans la formule (12b) ; R₁₇ représente H, un groupe alkyle en C₁ à C₁₂, alcoxy en C₁ à C₁₂, N-alkyle en C₁ à C₁₂-amino ou -NR₂₅R₂₆ dans lequel R₂₅ est un groupe alkyle en C₁ à C₈ et R₂₆ représente H ou un groupe alkyle en C₁ à C₈ ; R₁₈ est un groupe alkyle en C₁ à C₈ linéaire ou ramifié, alcényle en C₂ à C₈ ou aryle en C₆ à C₁₀-alkyle en C₁ à C₈ ; R₁₉, indépendamment de R₁₈, a les mêmes définitions que R₁₈ ou est un groupe aryle en C₆ à C₁₀, ou bien R₁₈ et R₁₉ représentent ensemble un groupe -(CH₂)ₑ- où e est un nombre entier de 2 à 6 ; R₂₀ et R₂₁ représentent chacun indépendamment l'un de l'autre un groupe alkyle en C₁ à C₈ linéaire ou ramifié qui peut être substitué par un groupe alcoxy en C₁ à C₄, ou aryle en C₆ à C₁₀-alkyle en C₁ à C₈ ou alcényle en C₂ à C₈ ; ou bien R₂₀ et R₂₁ représentent ensemble un groupe -(CH₂)_{f1}-Z₃-(CH₂)_{f2}- dans lequel Z₃ est une liaison directe, -O-, -S- ou -NR₂₆-, et R₂₆ représente H ou un groupe alkyle en C₁ à C₈ et f1 et f2 représentent chacun indépendamment l'un de l'autre un nombre entier de 2 à 4 ; R₂₃ et R₂₄ représentent chacun indépendamment l'un de l'autre H, un groupe alkyle en C₁ à C₈, cycloalkyle en C₃ à C₈; benzyle ou phényle ; et a, b1, b2 et b3 ont indépendamment l'un de l'autre une valeur de 0 ou 1 ; sous réserve que b1 et b2 aient chacun une valeur de 0 lorsque R₁₅ représente H ; que la somme (b1+b2+b3) ne dépasse pas 2 ; et que a ait une valeur de 0 lorsque R₁₂ est une liaison directe.

11. Matériau composite selon l'une quelconque des revendications 1 à 10, dans lequel conformément à (b), on applique un macromonomère hydrophile de formule : 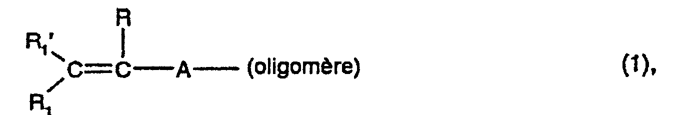 où R₁ représente l'hydrogène, un groupe alkyle en C₁ à C₆ ou un radical -COOR' ;
R, R' et R'₁ représentent chacun indépendamment les uns des autres l'hydrogène ou un groupe alkyle en C₁ à C₆ ;
A est une liaison directe ou est un radical de formule :
-C(O)-(A₁)ₙ-X- (2a)
ou
-(A₂)ₘ-NH-C(O)-X- (2b)
ou
-(A₂)ₘ-X-C(O)- (2c)
ou
-C(O)-NH-C(O)-X- (2d)
ou
-C(O)-X₁-(alk*)-X-C(O)- (2e)
ou
A et R₁, conjointement avec la double liaison adjacente, représentent un radical de formule : 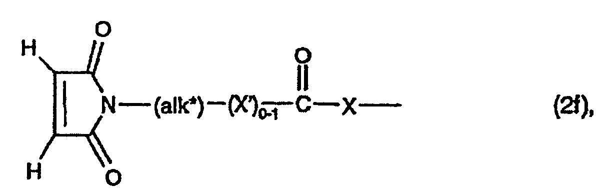 A₁ est un groupe -O-alkylène en C₂ à C₁₂ qui est non substitué ou substitué par un groupe hydroxy, ou est un groupe -O-alkylène en C₂ à C₁₂-NH-C(O)- ou -O-alkylène en C₂ à C₁₂-O-C(O)-NH-R₁₁-NH-C(O)-, où R₁₁ est un groupe alkylène en C₁ à C₁₈ linéaire ou ramifié ou arylène en C₆ à C₁₀ non substitué ou substitué par un groupe alkyle en C₁ à C₄ ou alcoxy en C₁ à C₄, aralkylène en C₇ à C₁₈, arylène en C₆ à C₁₀-alkylène en C₁ ou C₂-arylène en C₆ à C₁₀, cycloalkylène en C₃ à C₈, cycloalkylène en C₃ à C₈-alkylène en C₁ à C₆, cycloalkylène en C₃ à C₈-alkylène en C₁ ou C₂-cycloalkylène en C₃ à C₈ ou alkylène en C₁ à C₆-cycloalkylène en C₃ à C₈-alkylène en C₁ à C₆ ;
A₂ est un groupe alkylène en C₁ à C₈ ; phénylène ou benzylène ;
m et n représentent chacun indépendamment l'un de l'autre le nombre 0 ou 1;
X, X₁ et X' représente chacun indépendamment les uns des autres un groupe -O- bivalent ou -NR", dans lequel R" représente l'hydrogène ou un groupe alkyle en C₁ à C₆ ;
(alk*) est un groupe alkylène en C₂ à C₁₂ ;
et (oligomère) désigne :
(i) le radical d'un télomère de formule : 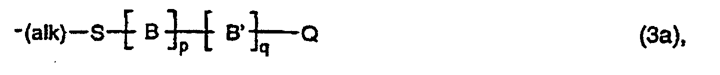 dans laquelle
(alk) est un groupe alkylène en C₂ à C₁₂,
Q est un groupe monovalent approprié pour jouer le rôle d'un terminateur de réaction en chaîne de polymérisation,
p et q représentent chacun indépendamment l'un de l'autre un nombre entier de 0 à 250, la somme (p+q) étant un nombre entier de 2 à 250,
et B et B' représentent chacun indépendamment l'un de l'autre un radical 1,2-éthylène pouvant être dérivé d'un monomère vinylique copolymérisable en remplaçant la double liaison vinyle par une simple liaison, au moins l'un des radicaux B et B' étant substitué par un substituant hydrophile ; ou bien
(ii) le radical d'un oligomère de formule : 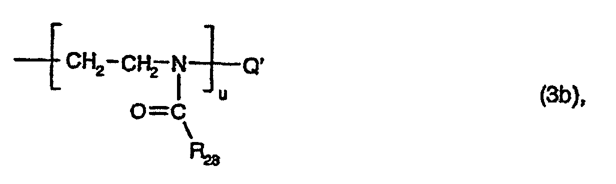 dans laquelle R₂₈ représente l'hydrogène ou un groupe alkyle en C₁ à C₁₂ non substitué ou à substitution hydroxy, u est un nombre entier de 2 à 250, et Q' est un radical d'un initiateur de polymérisation ; ou bien
(iii) le radical de formule : 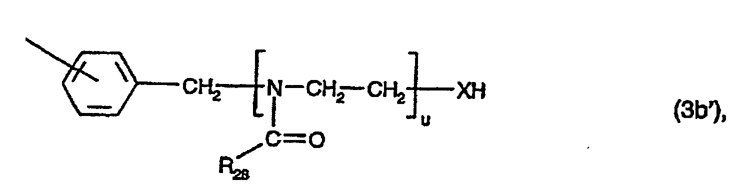 dans laquelle R₂₈, X et u sont tels que définis ci-dessus, ou
(iv) le radical d'un oligomère de formule : 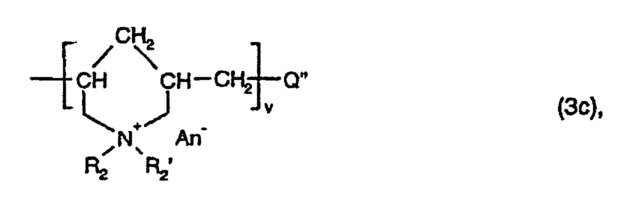 dans laquelle R₂ et R'₂ représentent chacun indépendamment un groupe alkyle en C₁ à C₄, An⁻ est un anion, v est un nombre entier de 2 à 250, et Q" est un groupe monovalent approprié pour jouer le rôle d'un terminateur de réaction en chaîne de polymérisation ; ou
(v) le radical d'un oligopeptide de formule :
-(CHR₄-C(O)-NH)ₜ-CHR₄-COOH (3d)
ou
-CHR₄-(NH-C(O)-CHR₄)ₜ-NH₂ (3d')
où R₄ représente l'hydrogène ou un groupe alkyle en C₁ à C₄ qui est non substitué ou substitué par un groupe hydroxy, carboxy, carbamoyle, amino, phényle, o-, m- ou p-hydroxyphényle, imidazolyle, indolyle ou un radical -NH-C(=NH)-NH₂ et t est un nombre entier de 2 à 250, ou le radical d'un oligopeptide à base de proline ou d'hydroxyproline ; ou
(vi) le radical d'un polyalkylèneoxyde de formule :
-(alk"-O)_{z}-[CH₂-CH₂-O]ᵣ-[CH₂-CH(CH₃)-O]ₛ-R₃₀ (3e)
dans laquelle R₃₀ représente l'hydrogène ou un groupe alkyle en C₁ à C₂₄, (alk") est un groupe alkylène en C₂ à C₄, z vaut 0 ou 1, r et s représentent chacun indépendamment un nombre entier de 0 à 250 et la somme (r+s) a une valeur de 2 à 250 ; ou
(vii) le radical d'un oligosaccharide;
sous réserve que
A ne soit pas une liaison directe si (oligomère) est un radical de formule (3a) ;
A soit une liaison directe si (oligomère) est un radical de formule (3b') ;
A ne soit pas un radical de formule (2c) ou (2e) si (oligomère) est un radical de formule (3b), (3c), (3d), (3e) ou est le radical d'un oligosaccharide ; et
A soit un radical de formule (2c) ou (2e) si (oligomère) est un radical de formule (3d').

12. Matériau composite selon la revendication 11, dans lequel le macromonomère hydrophile est un composé de formule (1), dans laquelle R représente l'hydrogène ou un groupe méthyle, R₁ représente l'hydrogène, un groupe méthyle ou carboxyle, R'₁ représente l'hydrogène, A est un radical de formule (2a) ou (2b), et (oligomère) est le radical d'un télomère de formule (3a).

13. Matériau composite selon la revendication 11 ou 12, dans lequel (oligomère) désigne un radical de formule (3a), et le radical -(alk)-S-[B]ₚ-[B']_{q}-Q est un radical de formule : 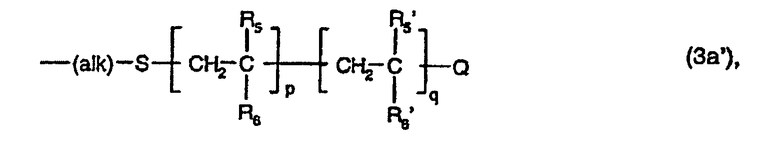 dans laquelle (alk) est un groupe alkylène en C₂ à C₄, R₅ et R'₅ représentent chacun indépendamment l'hydrogène ou un groupe méthyle, Q est un groupe monovalent approprié pour jouer le rôle d'un terminateur de réaction en chaîne de polymérisation, p et q représentent chacun indépendamment un nombre entier de 0 à 100, la somme (p+q) représentant un nombre entier de 5 à 100, et R₆ et R'₆ représentent chacun indépendamment un radical -COOY où Y est un groupe alkyle en C₁ ou C₂, alkyle en C₂ ou C₃, qui est substitué par un groupe hydroxy, amino ou N,N-di-alkyle en C₁ ou C₂-amino, ou est un radical -alkylène en C₂ à C₄-NH-C(O)-O-G où -O-G est le radical du tréhalose ; un radical -CO-NY₁Y₂, dans lequel Y₁ et Y₂ représentent chacun indépendamment l'un de l'autre l'hydrogène ou un groupe alkyle en C₁ ou C₂, qui est non substitué ou substitué par un groupe hydroxy, ou bien Y₁ et Y₂, conjointement avec l'atome de N adjacent, forment un cycle N-alkyle en C₁ ou C₂-pipérazino ou morpholino ; un radical hétérocyclique choisi dans le groupe constitué par les groupes N-pyrrolidonyle, 2- ou 4-pyridinyle, 2-méthylpyridin-5-yle, 2-, 3- ou 4-hydroxypyridinyle, N-ε-caprolactamyle, N-imidazolyle, 2-méthylimidazol-1-yle, N-morpholinyle et 4-N-méthylpipérazin-1-yle ; -COOH ; -SO₃H ; o-, m- ou p-sulfophényle ; o-, m- ou p-sulfométhylphényle ; un radical -CONY₅Y₆ dans lequel Y₅ est un groupe alkyle en C₂ à C₄ substitué par un groupe sulfo, et Y₆ représente l'hydrogène ; un groupe alkyle en C₁ à C₄ qui est substitué par un groupe -NR₉R'₉R"₉⁺An⁻ dans lequel R₉, R'₉ et R"₉ représentent chacun indépendamment l'un de l'autre l'hydrogène ou un groupe alkyle en C₁ à C₄ et An⁻ est un anion ; un radical -C(O)OY₇ dans lequel Y₇ est un groupe alkyle en C₂ à C₄, qui est substitué par un groupe -NR₉R'₉R"₉⁺An⁻ et est en outre non substitué ou substitué par un groupe hydroxy, dans lequel R₉, R'₉, R"₉⁺ et An⁻ sont tels que définis ; et un radical -C(O)-O-CH₂-CH(OY₈)-CH₂-O-PO₂⁻-(CH₂)₂-N(CH₃)₃⁺, dans lequel Y₈ représente l'hydrogène ou le radical acyle d'un acide gras supérieur.

14. Matériau composite selon l'une quelconque des revendications 11 à 13, dans lequel le macromonomère hydrophile appliqué conformément à (b) répond à la formule : 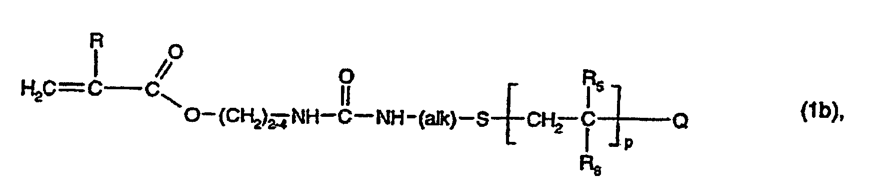 dans laquelle R représente l'hydrogène ou un groupe méthyle, (alk) est un groupe alkylène en C₂ à C₄, R₅ représente l'hydrogène ou un groupe méthyle, p est un nombre entier de 5 à 50, Q est tel que défini, et R₆ est un radical -CONH₂, -CON(CH₃)₂, -CONH-(CH₂)₂-OH, 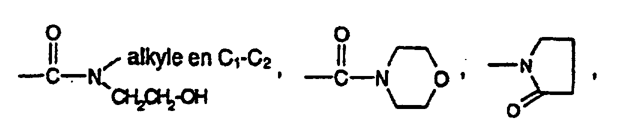 -COO-(CH₂)₂-N(CH₃)₂ ou -COO(CH₂)₂₋₄-NHC(O)-O-G, où -O-G est le radical du tréhalose.

15. Matériau composite selon l'une quelconque des revendications 1 à 14, dans lequel la polymérisation des monomères ou macromères à la surface du matériau en masse modifié est initiée par l'action d'une irradiation.

16. Matériau composite selon la revendication 15, dans lequel la polymérisation est initiée par l'action d'un rayonnement UV pendant une période de temps de 0,5 à 5 minutes.

17. Matériau composite selon l'une quelconque des revendications 1 à 16, dans lequel le revêtement hydrophile (b) de surface est susceptible d'être obtenu en greffant au moins un macromonomère à la surface du matériau en masse, avec formation d'une structure de type goupillon composée de chaînes dentées.

18. Matériau composite selon l'une quelconque des revendications 1 à 17, dans lequel le revêtement hydrophile de surface a une épaisseur de revêtement de 0,01 à 5,0 µm, et de préférence de 0,1 à 1 µm.

19. Procédé de fabrication d'un matériau composite, qui comprend les étapes consistant à :
(a) fournir un matériau en masse inorganique ou organique qui est dépourvu de groupes ioniques et présente une matière polyionique, adsorbée ou liée de manière hétéropolaire à sa surface, qui comprend des groupements initiateurs liés de manière covalente pour une polymérisation radicalaire ;
(b) appliquer un revêtement d'un ou plusieurs monomères ou macromonomères différents hydrophiles à insaturation éthylénique à la surface du matériau en masse pourvu des radicaux initiateurs, et
(c) polymériser le revêtement constitué de macromonomères hydrophiles insaturés, thermiquement ou par irradiation, de préférence par un rayonnement UV.

20. Procédé selon la revendication 19, dans lequel la matière polyionique comprend au moins une bicouche, la bicouche comprenant un premier polymère ionique et un second polymère ionique ayant des charges opposées aux charges du premier polymère ionique, ladite bicouche étant appliquée à la surface du matériau en masse par un procédé d'immersion comprenant les étapes consistant à (i) immerger le matériau en masse dans une solution du premier polymère ionique ; puis (ii) à immerger le matériau en masse dans une solution du second polymère ionique ayant des charges opposées aux charges du premier polymère ionique.

21. Procédé selon la revendication 20, dans lequel le premier polymère ionique est un polymère anionique comprenant des groupes carboxy ou un de ses sels, et le second polymère ionique est un polymère cationique comprenant des groupes amino primaires ou secondaires, ou un de ses sels.

22. Procédé selon l'une quelconque des revendications 19 à 21, dans lequel les groupements initiateurs pour la polymérisation radicalaire sont liés à la matière polyionique par réaction des groupes amino de la matière polyionique avec des groupes isocyanato du groupement initiateur.

23. Procédé selon l'une quelconque des revendications 19 à 22, dans lequel le matériau en masse inorganique ou organique est un dispositif biomédical, en particulier une lentille de contact, une lentille intraoculaire ou une cornée artificielle.

24. Dispositif biomédical comprenant un matériau composite selon l'une quelconque des revendications 1 à 18.

25. Dispositif biomédical selon la revendication 24, dans lequel le dispositif biomédical est une lentille de contact, une lentille intraoculaire ou une cornée artificielle.

26. Utilisation d'un matériau composite selon l'une quelconque des revendications 1 à 18 pour la fabrication d'un dispositif ophtalmique, en particulier pour la fabrication d'une lentille de contact, d'une lentille intraoculaire ou d'une cornée artificielle.
